(19) Europäisches Patentamt
European Patent Office
Office européen des brevets

(11) EP 4 545 642 A1

# (12) EUROPEAN PATENT APPLICATION
published in accordance with Art. 153(4) EPC

(43) Date of publication:
**30.04.2025 Bulletin 2025/18**

(21) Application number: **23846536.3**

(22) Date of filing: **25.07.2023**

(51) International Patent Classification (IPC):
***C12N 15/53*** *(2006.01)* ***C12N 1/15*** *(2006.01)*
***C12N 1/19*** *(2006.01)* ***C12N 1/21*** *(2006.01)*
***C12N 5/10*** *(2006.01)* ***C12N 9/02*** *(2006.01)*
***C12N 9/04*** *(2006.01)* ***C12N 15/63*** *(2006.01)*
***C12P 7/22*** *(2006.01)*

(52) Cooperative Patent Classification (CPC):
**C12N 5/10; C12N 9/0004; C12N 15/63; C12N 15/74; C12N 15/80; C12N 15/81; C12P 7/22**

(86) International application number:
**PCT/JP2023/027278**

(87) International publication number:
**WO 2024/024806 (01.02.2024 Gazette 2024/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.07.2022 JP 2022120869**
**30.09.2022 JP 2022158419**

(71) Applicant: **TOKYO UNIVERSITY OF SCIENCE FOUNDATION**
**Tokyo 162-8601 (JP)**

(72) Inventors:
- **FURUYA, Toshiki**
  **Tokyo 162-8601 (JP)**
- **SAKAMOTO, Satsuki**
  **Tokyo 162-8601 (JP)**
- **FUJIMAKI, Shizuka**
  **Tokyo 162-8601 (JP)**

(74) Representative: **Isarpatent**
**Patent- und Rechtsanwälte**
**Barth Hassa Peckmann & Partner mbB**
**Friedrichstraße 31**
**80801 München (DE)**

Remarks:
The complete document including Reference Table(s) and the Sequence Listing(s) can be downloaded from the EPO website

## (54) PHENOLIC COMPOUND PRODUCTION METHOD, AND MODIFIED ENZYME

(57) A method of producing a phenolic compound, the method producing a compound represented by the following Formula (2) from a compound represented by the following Formula (1), the method including a process of contacting the compound represented by Formula (1) with an enzyme, the enzyme having an activity to cleave a carbon-carbon double bond in the compound represented by Formula (1), and the method excluding a case in which the compound represented by Formula (1) is ferulic acid and the enzyme is a protein having an amino acid sequence represented by SEQ ID NO: 1. In the Formulas, $R^1$ represents a hydrogen atom or an alkyl group, each $R^2$ independently represents an alkoxy group, a hydroxy group, an alkyl group or a halogen atom, and n is a number from 0 to 4.

EP 4 545 642 A1

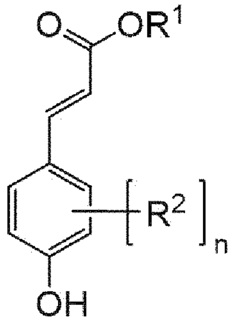

(1)

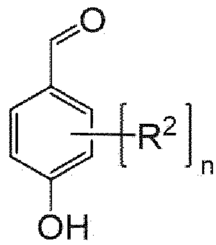

(2)

## Description

Technical Field

The present disclosure relates to a method of producing a phenolic compound, and a modified enzyme.

Background Art

Vanillin, which is used as a fragrance for food and beverage products or cosmetic products or as an intermediate for medical products, is commercially available not only as an extract obtained from a plant that belongs to the genus Vanilla in the orchid family but also as a chemically synthesized product. In view of a recent increase in interest in natural products and the SDGs, demands for vanillin produced without using petroleum or chemicals have been growing. Therefore, a method of producing vanillin with the aid of microorganisms or enzymes has been proposed. For example, a method of producing vanillin from ferulic acid, which can be obtained from agricultural wastes such as rice bran or wheat bran, using an enzymatic reaction, has been reported.

For example, Non-Patent Document 1 and Non-Patent Document 2 report a method of synthesizing vanillin via a first enzymatic reaction to convert ferulic acid as a starting material to 4-vinyl guaiacol as an intermediate, and a second enzymatic reaction to convert the intermediate to vanillin. Non-Patent Document 3 reports a method of synthesizing vanillin via a first enzymatic reaction to convert ferulic acid as a starting material to feruloyl-coenzyme A as an intermediate, and a second enzymatic reaction to convert the intermediate to vanillin. Non-Patent Document 4 reports a method in which ferulic acid is converted to vanillin using a plant-based hydrolysis enzyme that belongs to the cysteine protease family.

Non-Patent Document 1: J Am Chem Soc, 140, 16001-16005 (2018)
Non-Patent Document 2: ChemBioChem, 15, 2248-2254 (2014)
Non-Patent Document 3: Biotechnol Lett, 27, 1829-1832 (2005)
Non-Patent Document 4: Nat Commun, 5, 4037 (2014)

Summary of the Invention

Problem to be Solved by the Invention

The methods described in Non-Patent Documents 1 to 3, in which two steps of reactions are performed using two kinds of enzymes, have room for improvement in production efficiency.

Non-Patent Document 4 describes an enzymatic activity to convert ferulic acid to vanillin only in a qualitative manner without showing quantitative results, due to the weak activity of the enzyme used for the reaction.

In view of the foregoing, the present disclosure aims to provide a novel method of producing a phenolic compound by means of enzymatic reaction, and a novel enzyme that can be used for the production of a phenolic compound.

[Means for Solving the Problem]

The means for solving the foregoing problem includes the following embodiments.

<1> A method of producing a phenolic compound, the method producing a compound represented by the following Formula (2) from a compound represented by the following Formula (1):

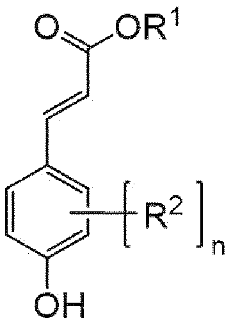
(1)

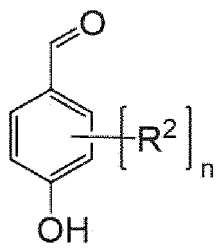
(2)

wherein, in the Formulas, $R^1$ represents a hydrogen atom or an alkyl group, each $R^2$ independently represents an

alkoxy group, a hydroxy group, an alkyl group or a halogen atom, and n is a number from 0 to 4,
the method including a process of contacting the compound represented by Formula (1) with an enzyme,
the enzyme having an activity to cleave a carbon-carbon double bond in the compound represented by Formula (1), and
the method excluding a case in which the compound represented by Formula (1) is ferulic acid and the enzyme is a protein having an amino acid sequence represented by SEQ ID NO: 1.

<2> The method of producing a phenolic compound according to <1>, wherein the enzyme is a cleavage oxygenase that acts on a compound having a carbon-carbon double bond as a substrate.
<3> The method of producing a phenolic compound according to <1> or <2>, wherein the enzyme is a protein having an amino acid sequence that satisfies at least one condition selected from the following (a1) to (a6) and (b):

(a1) an amino acid residue at an 82nd position in an amino acid sequence represented by SEQ ID NO: 1 is an amino acid residue other than a phenylalanine residue;
(a2) an amino acid residue at a 124th position in the amino acid sequence represented by SEQ ID NO: 1 is an amino acid residue other than a tyrosine residue;
(a3) an amino acid residue at a 157th position in the amino acid sequence represented by SEQ ID NO: 1 is an amino acid residue other than a lysine residue;
(a4) an amino acid residue at a 332nd position in the amino acid sequence represented by SEQ ID NO: 1 is an amino acid residue other than a valine residue;
(a5) an amino acid residue at a 333rd position in the amino acid sequence represented by SEQ ID NO: 1 is an amino acid residue other than a phenylalanine residue;
(a6) an amino acid residue at a 334th position in the amino acid sequence represented by SEQ ID NO: 1 is an amino acid residue other than a phenylalanine residue; and
(b) having an amino acid sequence identity of 70% or more with respect to an amino acid sequence that satisfies a condition selected from (a1) to (a6), expect for the amino acid sequence represented by SEQ ID NO: 1.

<4> The method of producing a phenolic compound according to <3>, wherein the enzyme is a protein having an amino acid sequence that satisfies a condition of (a1) and at least one condition selected from (a2) to (a6), or a protein having an amino acid sequence identity of 70% or more with respect to the amino acid sequence.
<5> The method of producing a phenolic compound according to <3> or <4>, wherein in (a1), the amino acid residue at the 82nd portion is a tyrosine residue or a triptophan residue.
<6> The method of producing a phenolic compound according to any one of <3> to <5>, wherein in (a4), (a5) or (a6), at least one amino acid residue at a position selected from the 332nd portion, the 333rd position or the 334 position is a polar amino acid residue.
<7> The method of producing a phenolic compound according to any one of <1> to <6>, wherein the compound represented by Formula (1) is ferulic acid or a ferulic acid alkyl ester.
<8> The method of producing a phenolic compound according to any one of <1> to <7>, wherein the compound represented by Formula (2) is vanillin or ethyl vanillin.
<9> A method of producing a phenolic compound, the method producing a compound represented by the following Formula (4) from a compound represented by the following Formula (3):

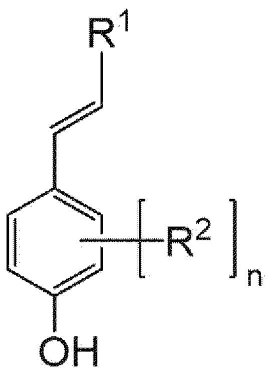
(3)

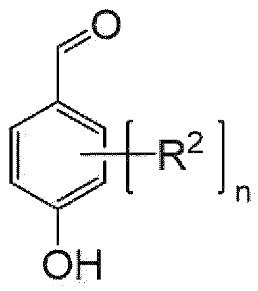
(4)

wherein, in the Formulas, $R^1$ represents a hydrogen atom, an alkyl group or $COOR^3$, each $R^2$ independently represents an alkoxy group, a hydroxy group, an alkyl group or a halogen atom, $R^3$ represents a hydrogen atom or an alkyl group, and n is a number from 0 to 4,

the method including a process of contacting the compound represented by Formula (3) with an enzyme, and the enzyme having an activity to cleave a carbon-carbon double bond in the compound represented by Formula (3) and being a protein having an amino acid sequence that satisfies at least one condition selected from the following (a1) to (a6) and (b):

(a1) an amino acid residue at an 82nd position in an amino acid sequence represented by SEQ ID NO: 1 is an amino acid residue other than a phenylalanine residue;
(a2) an amino acid residue at a 124th position in the amino acid sequence represented by SEQ ID NO: 1 is an amino acid residue other than a tyrosine residue;
(a3) an amino acid residue at a 157th position in the amino acid sequence represented by SEQ ID NO: 1 is an amino acid residue other than a lysine residue;
(a4) an amino acid residue at a 332nd position in the amino acid sequence represented by SEQ ID NO: 1 is an amino acid residue other than a valine residue;
(a5) an amino acid residue at a 333rd position in the amino acid sequence represented by SEQ ID NO: 1 is an amino acid residue other than a phenylalanine residue;
(a6) an amino acid residue at a 334th position in the amino acid sequence represented by SEQ ID NO: 1 is an amino acid residue other than a phenylalanine residue; and
(b) having an amino acid sequence identity of 70% or more with respect to an amino acid sequence that satisfies a condition selected from (a1) to (a6), expect for the amino acid sequence represented by SEQ ID NO: 1.

<10> A modified enzyme, having an activity to cleave a carbon-carbon double bond in a phenolic compound, and being a protein having an amino acid sequence that satisfies at least one condition selected from the following (a1) to (a6) and (b):

(a1) an amino acid residue at an 82nd position in an amino acid sequence represented by SEQ ID NO: 1 is an amino acid residue other than a phenylalanine residue;
(a2) an amino acid residue at a 124th position in the amino acid sequence represented by SEQ ID NO: 1 is an amino acid residue other than a tyrosine residue;
(a3) an amino acid residue at a 157th position in the amino acid sequence represented by SEQ ID NO: 1 is an amino acid residue other than a lysine residue;
(a4) an amino acid residue at a 332nd position in the amino acid sequence represented by SEQ ID NO: 1 is an amino acid residue other than a valine residue;
(a5) an amino acid residue at a 333rd position in the amino acid sequence represented by SEQ ID NO: 1 is an amino acid residue other than a phenylalanine residue;
(a6) an amino acid residue at a 334th position in the amino acid sequence represented by SEQ ID NO: 1 is an amino acid residue other than a phenylalanine residue; and
(b) having an amino acid sequence identity of 70% or more with respect to an amino acid sequence that satisfies a condition selected from (a1) to (a6), expect for the amino acid sequence represented by SEQ ID NO: 1.

<11> The modified enzyme according to <10>, which is used for a method of producing a phenolic compound.
<12> A composition, including the modified enzyme according to <10> or <11>.
<13> A polynucleotide, encoding the modified enzyme according to <10>.
<14> An expression vector, including the polynucleotide according to <13>.
<15> A transformant, including the expression vector according to <14>.

[Effect of the Invention]

According to the present disclosure, a novel method of producing a phenolic compound by means of enzymatic reaction, and a novel enzyme that can be used for the production of a phenolic compound are provided.

[Brief Explanation of the Drawings]

FIG. 1 is diagrams illustrating the absorption spectra of the product obtained in Example 1.
FIG. 2 is a graph illustrating the results of quantification of the product obtained in Example 1.
FIG. 3 is a graph illustrating the results of quantification of the product obtained in Example 2.

FIG. 4 is a graph illustrating the results of quantification of the product obtained in Example 3.
FIG. 5 is a graph illustrating the results of quantification of the product obtained in Example 4.
FIG. 6 is a graph illustrating the results of quantification of the product obtained in Example 5.
FIG. 7 is a graph illustrating the results of quantification of the product obtained in Example 6.
FIG. 8 is a graph illustrating the results of quantification of the product obtained in Example 7.
FIG. 9 is a graph illustrating the results of quantification of the product obtained in Example 8.
FIG. 10 is a graph illustrating the results of quantification of the product obtained in Example 9.
FIG. 11 is a graph illustrating the results of quantification of the product obtained in Example 10.

[Embodiments for Implementing the Invention]

In the following, the embodiments of the present disclosure are described. The explanation and the examples described therein are directed to exemplify the present invention, rather than restricting the scope of the present invention.
The numeric ranges described in the specification using the expression "from ... to ..." represents a range in which numerical values described before and after the "to" are included in the range as a minimum value and a maximum value, respectively.

<First Embodiment>

The first embodiment of the present disclosure is a method of producing a phenolic compound, the method producing a compound represented by the following Formula (2) from a compound represented by the following Formula (1),

the method including a process of contacting the compound represented by Formula (1) with an enzyme, and the enzyme having an activity to cleave a carbon-carbon double bond in the compound represented by Formula (1), wherein the method excludes a case in which the compound represented by Formula (1) is ferulic acid and the enzyme is a protein having an amino acid sequence represented by SEQ ID NO: 1.

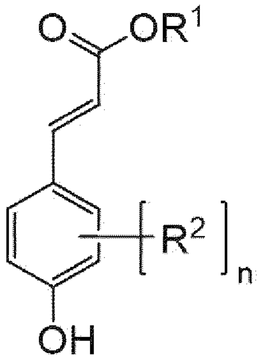 (1)

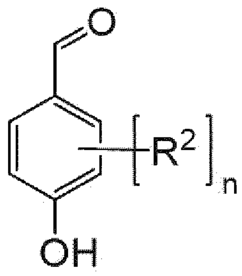 (2)

In the Formulas, $R^1$ represents a hydrogen atom or an alkyl group, each $R^2$ independently represents an alkoxy group, a hydroxy group, an alkyl group or a halogen atom, and n is a number from 0 to 4.
According to the foregoing method, it is possible to produce a compound represented by Formula (2) (for example, vanillin) from a compound represented by Formula (1) (for example, ferulic acid) without going through the production of an intermediate. Therefore, the method excels in productivity as compared with a method in which a compound represented by Formula (2) is produced from a compound represented by Formula (1) through the production of an intermediate.
Further, the foregoing method can be carried out without using a coenzyme, and therefore has an advantage in terms of reducing the production cost.
In the following, an enzyme having an activity to cleave a carbon-carbon double bond of the compound represented by Formula (1) is also referred to as the specific enzyme.
The specific enzyme has an activity to cleave a carbon-carbon double bond, which binds the aromatic ring with $COOR^1$ in the compound represented by Formula (1), in the presence of molecular oxygen ($O_2$). Specifically, the specific enzyme catalyzes the following reaction.

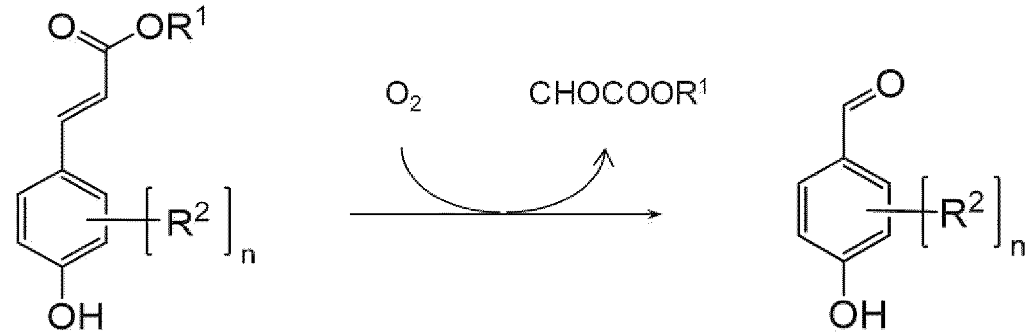

The specific enzyme is preferably a cleavage oxygenase that acts on a compound having a carbon-carbon double bond as a substrate.

Specific examples of the cleavage oxygenase include carotenoid cleavage oxygenase, stilbene cleavage oxygenase, isoeugenol cleavage oxygenase, and 4-vinyl guaiacol cleavage oxygenase.

The specific enzyme may be an enzyme derived from a natural product, or may be an enzyme that is artificially modified or synthesized.

In an embodiment of the present embodiment, the specific enzyme is a protein having an amino acid sequence that satisfies at least one condition selected from the following (a1) to (a6) and (b):

(a1) an amino acid residue at an 82nd position in an amino acid sequence represented by SEQ ID NO: 1 is an amino acid residue other than a phenylalanine residue;
(a2) an amino acid residue at a 124th position in the amino acid sequence represented by SEQ ID NO: 1 is an amino acid residue other than a tyrosine residue;
(a3) an amino acid residue at a 157th position in the amino acid sequence represented by SEQ ID NO: 1 is an amino acid residue other than a lysine residue;
(a4) an amino acid residue at a 332nd position in the amino acid sequence represented by SEQ ID NO: 1 is an amino acid residue other than a valine residue;
(a5) an amino acid residue at a 333rd position in the amino acid sequence represented by SEQ ID NO: 1 is an amino acid residue other than a phenylalanine residue;
(a6) an amino acid residue at a 334th position in the amino acid sequence represented by SEQ ID NO: 1 is an amino acid residue other than a phenylalanine residue; and
(b) having an amino acid sequence identity of 70% or more with respect to an amino acid sequence that satisfies a condition selected from (a1) to (a6), expect for the amino acid sequence represented by SEQ ID NO: 1.

The protein having an amino acid sequence represented by SEQ ID NO: 1 is an oxygenase derived from a fungus, Thermothelomyces thermophila (hereinafter, also referred to as Ado, Gen BANK accession No. XP_003665585).

Non-patent document 1 describes that Ado has an activity to convert 4-vinyl guaiacol or isoeugenol, which is an intermediate obtained by converting ferulic acid as a starting material, to vanillin. However, there has been no report on the case in which a starting material is directly converted by Ado to vanillin.

Further, as shown in the following examples, Ado is proved to exhibit little activity to convert ferulic acid to vanillin.

The present inventors have applied an enzyme, obtained by converting an amino acid residue at a specific site of Ado to a different amino acid residue, to the method of converting the compound represented by Formula (1) to the compound represented by Formula (2). As a result, the present inventors have proved an increase in the activity of Ado to convert the compound represented by Formula (1) to vanillin.

The reason for the foregoing results is thought to be that, for example, the amino acid residue that has been modified at a specific site of Ado functions as an active center that involves in the cleavage of the carbon-carbon double bond included in a phenol compound.

Further, it has been proved that the enzyme, obtained by converting an amino acid residue at a specific site of Ado to a different amino acid residue, exhibits an activity to convert the compound represented by Formula (1) to vanillin even in a case in which the compound represented by Formula (1) is ferulic acid.

The specific enzyme is a protein in which an amino acid residue is converted at least at the 82nd position, 124th position, 157th position, 332nd position, 333rd position or 334th position in the amino acid sequence represented by SEQ ID NO: 1.

In the amino acid sequence represented by SEQ ID NO: 1, the number of the amino acid residue to be converted is not particularly limited as long as it is 1 or more.

From the viewpoint of conversion efficiency for the compound represented by Formula (1), the number of the

amino acid residue to be converted in the amino acid sequence represented by SEQ ID NO: 1 is preferably 100 or less, more preferably 50 or less, further preferably 20 or less, particularly preferably 10 or less.

In an embodiment, the specific enzyme may be a protein in which an amino acid residue is converted at the 82nd position and at one or more of the 124th position, 157th position, 332nd position, 333rd position or 334th position, in the amino acid sequence represented by SEQ ID NO: 1.

The type of the amino acid residue that substitutes the amino acid residue at the 82nd position in the amino acid sequence represented by SEQ ID NO: 1 is not particularly limited. From the viewpoint of conversion efficiency for the compound represented by Formula (1), the amino acid residue at the 82nd position is preferably an aromatic amino acid residue, more preferably a tyrosine residue or a tryptophan residue.

The type of the amino acid residue that substitutes the amino acid residue at the 332nd position, 333rd position or 334th position in the amino acid sequence represented by SEQ ID NO: 1 is not particularly limited. From the viewpoint of conversion efficiency for the compound represented by Formula (1), at least one selected from the amino acid residues at the 332nd position, 333rd position or 334th position is preferably a residue of a polar amino acid (such as a basic amino acid or a hydroxy amino acid), more preferably a residue of a basic amino acid (arginine, lysine or histidine), further preferably a residue of arginine.

Specific examples of the specific enzyme include a protein having an amino acid sequence represented by SEQ ID NO: 2, in which the amino acid residue at the 82nd position in the amino acid sequence represented by SEQ ID NO: 1 is a tyrosine residue Y (hereinafter, also referred to as Ado-F82Y);

a protein having an amino acid sequence represented by SEQ ID NO: 3, in which the amino acid residue at the 82nd position in the amino acid sequence represented by SEQ ID NO: 1 is a tryptophan residue W (hereinafter, also referred to as Ado-F82W);

a protein having an amino acid sequence represented by SEQ ID NO: 4, in which the amino acid residue at the 82nd position is a tyrosine residue Y, and further the amino acid residue at the 332nd position is an amino acid residue X other than a valine residue, the amino acid residue at the 333rd position is an amino acid residue X other than a phenylalanine residue, or the amino acid residue at the 334th position is an amino acid residue X other than a phenylalanine residue, in the amino acid sequence represented by SEQ ID NO: 1 (hereinafter, also referred to as Ado-F82Y-V332X-F333X-F334X); and

a protein having an amino acid sequence identity of 70% or more with respect to the amino acid sequence represented by SEQ ID NO: 1, SEQ ID NO: 2 or SEQ ID NO: 3.

(SEQ ID NO: 1: Amino acid sequence of Ado)

MAHIHDLAPEVSNYSSGRLTPPTPVRFPRTPVFASMNKPCRFEGDVFDLEVSGAIP

PDIDGTFFRVQPDHRFPPLFEDDIHFNGDGSVTAIRISGGHADLRQRYVRTERYLLETRARR

SLFGRYRNPWTDNESVRGVIRTASNTNVVFWRGALLAMKEDGPPFAMDPVTLETLGRYD

FEGQILSPTFTAHPKIDPDTGEMVCFAYEAGGDGSDCSVDVAVWTVDADGKKVEECWYK

APFAGMIHDCGITKNWVVLPLTPIKMDLERMKRGGNKFAWDPSEDQWYGVVPRRGAKS

DDIIWFRADNGFHGHVAGCYELPSGEIVFDLTVADGNVFFFFPPDDNITPPADGVAKRNRL

SSPTVRWIFDPKAKKSAIRTEAAGDADIWVADERVKPALTWPTNGEFSRIDDRYVTKPYR

HFWQAVVDPTRPYDFEKCGPPAGGLFNCLGHYTWSDQNYHHGHNTGDPSGDGRSNGSA

EEATAGKFGLQDVYFAGPTMTFQEPTFIPRQGAAEGEGYLIALLNHLDELRNDVVIFEARN

LGKGPLAVIHLPLKLKLGLHGNWVDSREIEAWRRRRAENGDVGPLRVAKEPLPWQKKFA

AAAQNGSNGV*

(SEQ ID NO: 2: Amino acid sequence of Ado-F82Y)

MAHIHDLAPEVSNYSSGRLTPPTPVRFPRTPVFASMNKPCRFEGDVFDLEVSGAIP PDIDGTFFRVQPDHRFPPLFEDDIHYNGDGSVTAIRISGGHADLRQRYVRTERYLLETRARR SLFGRYRNPWTDNESVRGVIRTASNTNVVFWRGALLAMKEDGPPFAMDPVTLETLGRYD FEGQILSPTFTAHPKIDPDTGEMVCFAYEAGGDGSDCSVDVAVWTVDADGKKVEECWYK APFAGMIHDCGITKNWVVLPLTPIKMDLERMKRGGNKFAWDPSEDQWYGVVPRRGAKS DDIIWFRADNGFHGHVAGCYELPSGEIVFDLTVADGNVFFFFPPDDNITPPADGVAKRNRL SSPTVRWIFDPKAKKSAIRTEAAGDADIWVADERVKPALTWPTNGEFSRIDDRYVTKPYR HFWQAVVDPTRPYDFEKCGPPAGGLFNCLGHYTWSDQNYHHGHNTGDPSGDGRSNGSA EEATAGKFGLQDVYFAGPTMTFQEPTFIPRQGAAEGEGYLIALLNHLDELRNDVVIFEARN LGKGPLAVIHLPLKLKLGLHGNWVDSREIEAWRRRRAENGDVGPLRVAKEPLPWQKKFA AAAQNGSNGV*

(SEQ ID NO: 3: Amino acid sequence of Ado-F82Y)

MAHIHDLAPEVSNYSSGRLTPPTPVRFPRTPVFASMNKPCRFEGDVFDLEVSGAIP PDIDGTFFRVQPDHRFPPLFEDDIHWNGDGSVTAIRISGGHADLRQRYVRTERYLLETRAR RSLFGRYRNPWTDNESVRGVIRTASNTNVVFWRGALLAMKEDGPPFAMDPVTLETLGRY DFEGQILSPTFTAHPKIDPDTGEMVCFAYEAGGDGSDCSVDVAVWTVDADGKKVEECWY KAPFAGMIHDCGITKNWVVLPLTPIKMDLERMKRGGNKFAWDPSEDQWYGVVPRRGAK SDDIIWFRADNGFHGHVAGCYELPSGEIVFDLTVADGNVFFFFPPDDNITPPADGVAKRNR LSSPTVRWIFDPKAKKSAIRTEAAGDADIWVADERVKPALTWPTNGEFSRIDDRYVTKPYR HFWQAVVDPTRPYDFEKCGPPAGGLFNCLGHYTWSDQNYHHGHNTGDPSGDGRSNGSA EEATAGKFGLQDVYFAGPTMTFQEPTFIPRQGAAEGEGYLIALLNHLDELRNDVVIFEARN LGKGPLAVIHLPLKLKLGLHGNWVDSREIEAWRRRRAENGDVGPLRVAKEPLPWQKKFA AAAQNGSNGV*

(SEQ ID NO: 4: Amino acid sequence of Ado-F82Y-V332X-F333X-F334X)

MAHIHDLAPEVSNYSSGRLTPPTPVRFPRTPVFASMNKPCRFEGDVFDLEVSGAIP PDIDGTFFRVQPDHRFPPLFEDDIHYNGDGSVTAIRISGGHADLRQRYVRTERYLLETRARR SLFGRYRNPWTDNESVRGVIRTASNTNVVFWRGALLAMKEDGPPFAMDPVTLETLGRYD

FEGQILSPTFTAHPKIDPDTGEMVCFAYEAGGDGSDCSVDVAVWTVDADGKKVEECWYK

APFAGMIHDCGITKNWVVLPLTPIKMDLERMKRGGNKFAWDPSEDQWYGVVPRRGAKS

DDIIWFRADNGFHGHVAGCYELPSGEIVFDLTVADGNXXXFFPPDDNITPPADGVAKRNRL

SSPTVRWIFDPKAKKSAIRTEAAGDADIWVADERVKPALTWPTNGEFSRIDDRYVTKPYR

HFWQAVVDPTRPYDFEKCGPPAGGLFNCLGHYTWSDQNYHHGHNTGDPSGDGRSNGSA

EEATAGKFGLQDVYFAGPTMTFQEPTFIPRQGAAEGEGYLIALLNHLDELRNDVVIFEARN

LGKGPLAVIHLPLKLKLGLHGNWVDSREIEAWRRRRAENGDVGPLRVAKEPLPWQKKFA

AAAQNGSNGV*

When the specific enzyme is a protein of (b), the amino acid sequence identity with respect to an amino acid sequence that satisfies a condition selected from (a1) to (a6) may be 75% or more, 80% or more, 85% or more, 85% or more or 90% or more, as long as it is 70% or more.

When the specific enzyme is a protein of (b), the amino acid sequence identity with respect to an amino acid sequence that satisfies a condition selected from (a1) to (a6) may be 75% or more, 80% or more, 85% or more, 85% or more or 90% or more, as long as it is 70% or more.

In the present disclosure, the amino acid sequence identity of the protein of (b) is represented by a percentage obtained by aligning the amino acid sequence of the protein, such that the number of coincident amino acid residues with the amino acid sequence that satisfies a condition selected from (a1) to (a6) is maximized, and dividing the total number of coincident amino acid residues by the total number of amino acid residues of the amino acid sequence of (b).

The search for the amino acid sequence identity may be performed using a search program known in the art, such as BLAST (Basic Local Alignment Search Tool).

The total number of the amino acid residues of the specific enzyme may be the same as the amino acid sequence represented by SEQ ID NO: 1 (603 amino acid residues) or may be different. The total number of the amino acid residues of the specific enzyme, given as Y, may satisfy the following formula. In the formula, each Z1 and Z2 independently represents a number from 0 to 10, preferably a number from 0 to 5, more preferably a number from 0 to 2.

$$603\text{-}Z1 \leq Y \leq 603+Z2$$

In the compound represented by Formula (1), $R^1$ represents a hydrogen atom or an alkyl group, each $R^2$ independently represents an alkoxy group, a hydroxy group, an alkyl group or a halogen atom, and n is a number from 0 to 4.

$R^1$ is preferably a hydrogen atom or an alkyl group with a carbon number from 1 to 5, more preferably a hydrogen atom or an alkyl group with a carbon number from 1 to 3, further preferably a hydrogen atom, a methyl group or an ethyl group.

Each $R^2$ preferably independently represents an alkoxy group with a carbon number from 1 to 5, a hydroxy group, an alkyl group with a carbon number from 1 to 5 or a halogen atom, more preferably an alkoxy group with a carbon number from 1 to 3, further preferably a methoxy group or an ethoxy group.

n is preferably a number from 0 to 2, more preferably 1.

When n is a number of 1 or more, at least one of $R^2$ is preferably at an ortho position with respect to the phenolic hydroxy group.

In the compound represented by Formula (2), each $R^2$ independently represents an alkoxy group, a hydroxy group, an alkyl group or a halogen atom, and n is a number from 0 to 4.

Each $R^2$ preferably independently represents an alkoxy group with a carbon number from 1 to 5, a hydroxy group, an alkyl group with a carbon number from 1 to 5 or a halogen atom, more preferably an alkoxy group with a carbon number from 1 to 3, further preferably a methoxy group or an ethoxy group.

n is preferably a number from 0 to 2, more preferably 1.

When n is a number of 1 or more, at least one of $R^2$ is preferably at an ortho position with respect to the phenolic hydroxy group.

Specific examples of the compound represented by Formula (1) include ferulic acid (a compound in which $R^1$ is a hydrogen atom, $R^2$ is a methoxy group, n is 1, and $R^2$ is at an ortho position with respect to the phenolic hydroxy group) and ferulic acid alkyl ester (a compound in which $R^1$ is an alkyl group, $R^2$ is a methoxy group, n is 1, and $R^2$ is at an ortho position

with respect to the phenolic hydroxy group).

Specific examples of the compound represented by Formula (2) include vanillin (a compound in which $R^2$ is a methoxy group, n is 1, and $R^2$ is at an ortho position with respect to the phenolic hydroxy group) and ethyl vanillin (a compound in which $R^2$ is an ethoxy group, n is 1, and $R^2$ is at an ortho position with respect to the phenolic hydroxy group).

When ferulic acid or ferulic acid alkyl ester is used as the compound represented by Formula (1), vanillin is obtained as the compound represented by Formula (2).

The enzyme used in the method of the present embodiment may be a single kind or two or more kinds.

The compound represented by Formula (1) used in the method of the present embodiment may be a single kind or two or more kinds.

The method for performing the process of contacting the compound represented by Formula (1) with the enzyme is not particularly limited. For example, the process may be performed in a reaction solution that contains the compound represented by Formula (1), the enzyme, and an aqueous medium.

The conditions for performing the process of contacting the compound represented by Formula (1) with the enzyme is not particularly limited. For example, the temperature of the reaction solution may be selected from a range of 10°C to 90°C, the pH of the reaction solution may be selected from a range of 2 to 13, and the reaction time may be selected from a range of 1 minute to 1 month.

The enzyme used in the method of the present embodiment may be in a state of a transformant that expresses the enzyme, and the like.

<Second Embodiment>

The second embodiment of the present disclosure is a method of producing a phenolic compound, the method producing a compound represented by the following Formula (4) from a compound represented by the following Formula (3),

the method including a process of contacting the compound represented by Formula (3) with an enzyme, and
the enzyme having an activity to cleave a carbon-carbon double bond in the compound represented by Formula (3) and being a protein having an amino acid sequence that satisfies at least one condition selected from the foregoing (a1) to (a6) and (b).

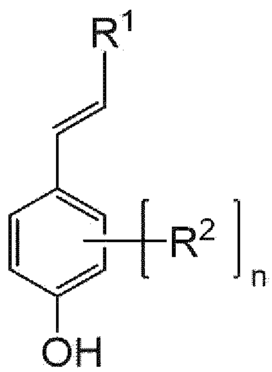

(3)

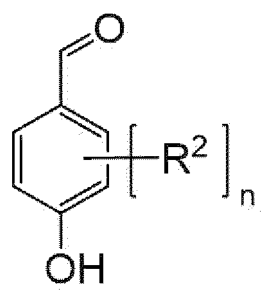

(4)

In the Formulas, $R^1$ represents a hydrogen atom, an alkyl group or $COOR^3$, each $R^2$ independently represents an alkoxy group, a hydroxy group, an alkyl group or a halogen atom, $R^3$ represents a hydrogen atom or an alkyl group, and n is a number from 0 to 4.

According to the foregoing method, a compound represented by Formula (4) can be produced from a compound represented by Formula (3).

Further, the foregoing method can be carried out without using a coenzyme, and therefore has an advantage in terms of reducing the production cost.

In the compound represented by Formula (3), $R^1$ is preferably a hydrogen atom, an alkyl group with a carbon number from 1 to 5, or $COOR^3$ where $R^3$ is a hydrogen atom or an alkyl group with a carbon number from 1 to 5; $R^1$ is more preferably a hydrogen atom, an alkyl group with a carbon number from 1 to 3, or $COOR^3$ where $R^3$ is a hydrogen atom or an alkyl group with a carbon number from 1 to 3; and $R^1$ is further preferably a hydrogen atom, a methyl group, an ethyl group, COOH, COOMe or COOEt.

Each of $R^2$ preferably independently represents an alkoxy group with a carbon number from 1 to 5, a hydroxy group, an alkyl group with a carbon number from 1 to 5 or a halogen atom; more preferably an alkoxy group with a carbon number from 1 to 3; further preferably a methoxy group or an ethoxy group.

n is preferably a number from 0 to 2, more preferably 1.

When n is a number of 1 or more, at least one of $R^2$ is preferably at an ortho position with respect to the phenolic hydroxy group.

In the compound represented by Formula (4), each $R^2$ preferably independently represents an alkoxy group with a carbon number from 1 to 5, a hydroxy group, an alkyl group with a carbon number from 1 to 5 or a halogen atom; more preferably an alkoxy group with a carbon number from 1 to 3; further preferably a methoxy group or an ethoxy group.

n is preferably a number from 0 to 2, more preferably 1.

When n is a number of 1 or more, at least one of $R^2$ is preferably at an ortho position with respect to the phenolic hydroxy group.

Specific examples of the compound represented by Formula (3) include ferulic acid (a compound in which $R^1$ is $COOR^3$, $R^2$ is a methoxy group, $R^3$ is a hydrogen atom, n is 1, and $R^2$ is at an ortho position with respect to the phenolic hydroxy group), ferulic acid alkyl ester (a compound in which $R^1$ is $COOR^3$, $R^2$ is a methoxy group, $R^3$ is an alkyl group, n is 1, and $R^2$ is at an ortho position with respect to the phenolic hydroxy group), 4-vinyl guaiacol (a compound in which $R^1$ is a hydrogen atom, $R^2$ is a methoxy group, n is 1, and $R^2$ is at an ortho position with respect to the phenolic hydroxy group) and isoeugenol (a compound in which $R^1$ is a methyl group, $R^2$ is a methoxy group, n is 1, and $R^2$ is at an ortho position with respect to the phenolic hydroxy group).

Specific examples of the compound represented by Formula (4) include vanillin (a compound in which $R^2$ is a methoxy group, n is 1, and $R^2$ is at an ortho position with respect to the phenolic hydroxy group) and ethyl vanillin (a compound in which $R^2$ is an ethoxy group, n is 1, and $R^2$ is at an ortho position with respect to the phenolic hydroxy group).

The enzyme used in the second embodiment is an enzyme that is encompassed in the specific enzyme used in the first embodiment, and is a protein having an amino acid sequence that satisfies the condition selected from (a1) to (a6) and (b).

The foregoing enzyme has an activity to cleave a carbon-carbon double bond, which binds the aromatic ring with $R^1$ in the compound represented by Formula (3), in the presence of molecular oxygen ($O_2$). Specifically, the specific enzyme catalyzes the following reaction.

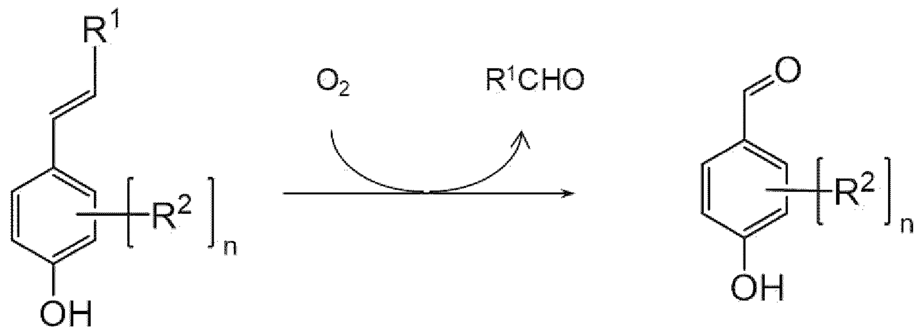


The details and preferred embodiments of the enzyme used in the method of the present embodiment are the same as the details and preferred embodiments of the enzyme used in the method of the first embodiment.

The enzyme used in the method of the present embodiment has, in addition to an activity to convert the compound represented by Formula (3) in which $R^1$ is $COOR^3$ to the compound represented by Formula (4), an activity to convert the compound represented by Formula (3) in which $R^1$ is a hydrogen atom or an alkyl group to the compound represented by Formula (4).

Further, the activity of the enzyme to convert the compound represented by Formula (3) to the compound represented by Formula (4) is superior to the activity of Ado to convert the compound represented by Formula (3) to the compound represented by Formula (4).

Therefore, the foregoing enzyme is usable also in a method including multiple steps, for example, a method in which a starting material such as ferulic acid is converted to an intermediate such as 4-vinyl guaiacol or isoeugenol, and then the intermediate is converted to a target material such as vanillin.

The enzyme used in the method of the present embodiment may be a single kind or two or more kinds.

The compound represented by Formula (3) used in the method of the present embodiment may be a single kind or two or more kinds.

The method for performing the process of contacting the compound represented by Formula (3) with the enzyme is not particularly limited. For example, the process may be performed in a reaction solution that contains the compound represented by Formula (3), the enzyme, and an aqueous medium.

The conditions for performing the process of contacting the compound represented by Formula (3) with the enzyme is not particularly limited. For example, the temperature of the reaction solution may be selected from a range of

10°C to 90°C, the pH of the reaction solution may be selected from a range of 2 to 13, and the reaction time may be selected from a range of 1 minute to 1 month.

The enzyme used in the method of the present embodiment may be in a state of a transformant that expresses the enzyme, and the like.

<Third Embodiment>

The third embodiment of the present disclosure is a modified enzyme, having an activity to cleave a carbon-carbon double bond in a phenolic compound, and being a protein having an amino acid sequence that satisfies at least one condition selected from the foregoing (a1) to (a6) and (b).

The details and preferred embodiments of the modified enzyme are the same as the details and preferred embodiments of the specific enzyme as described in the first embodiment.

The purposes of the modified enzyme include a method of producing a phenol compound (for example, the methods according to the first embodiment or the second embodiment as mentioned above).

The third embodiment includes a composition that includes the modified enzyme; a polynucleotide that encodes the modified enzyme; an expression vector that includes the modified enzyme; and a transformant that includes the expression vector.

The composition including the modified enzyme contains, for example, the modified enzyme and an aqueous medium. As necessary, the composition may contain a buffer, a pH adjuster, a surfactant, an organic solvent, and the like.

The modified enzyme included in the composition nay be in a state of a transformant that expresses the modified enzyme.

The polynucleotide that encodes the modified enzyme include a DNA and an RNA.

The expression vector that includes the polynucleotide encoding the modified enzyme may further include a region encoding a promoter, a terminator, a drug resistance gene or the like, in addition to the polynucleotide. The expression vector may be a plasmid or an integrative vector. The expression vector may be a virus vector or a vector for cell-free systems.

The transformant that includes the expression vector may be obtained by transforming a host using the expression vector, and culturing the host that has been transformed.

Examples of the host for introducing the expression vector include procaryotic cells such as bacteria in the genus Escherichia (such as Escherichia coli), the genus Corynebacterium or the genus Bacillus; and eukaryotic cells such as bacteria in the genus Saccharomyces, the genus Pichia or the genus Aspergillus. Among these, the host is preferably a bacterium in the genus Escherichia, more preferably Escherichia coli.

The medium used for the culture of the host may be selected from ordinary culture media, such as an LB medium.

It is possible to obtain a homogenate or a lysate including the modified enzyme by subjecting the transformant to a process such as cell homogenization or cell lysis.

[Examples]

In the following, the foregoing embodiments are explained by referring to the Examples. However, the embodiments are in no way restricted by the Examples.

[Preparation Example of Transformant 1]

The ado gene (SEQ ID NO: 5), encoding the oxidase Ado having an amino acid sequence represented by SEQ ID NO: 1, was prepared by chemical synthesis. The pETDado was prepared by performing PCR using the ado gene as a template, primers represented by SEQ ID NO: 6 and SEQ ID NO: 7 and KOD One PCR Master Mix (Toyobo), cleaving the amplified product using restriction enzymes (BamHI and NotI), and ligating the fragment with a pETDuet-1 vector (Novagen). The pETDado was introduced into E. coli BL21 Star (DE3) (Thermo Fisher), thereby preparing a recombinant E. coli. The recombinant E. coli was inoculated into an LB medium containing 50 μg/ml of ampicillin, and cultured for 6 hours at 30°C. Thereafter, isopropyl-β-D-thiogalactopyranoside was added at a final concentration of 0.1 mM and $FeCl_2$ was added at a final concentration of 1 mM, and cultured for 15 hours at 16°C to induce the expression of gene. The bacterial body was collected and washed with a phosphate buffer solution (137 mM NaCl, 2.7 mM KCl, 10 mM $Na_2HPO_4$, 2 mM $KH_2PO_4$, pH 7.4) and cryopreserved. An expression bacterial body of E. coli transduced with a gene encoding Ado was thus prepared.

[Preparation Example of Transformant 2]

The ado-F82Y gene (SEQ ID NO: 8), encoding the oxidase Ado-F82Y having an amino acid sequence

represented by SEQ ID NO: 2, was prepared by chemical synthesis. Specifically, the pETDado-F82Y, in which the ado-F82Y gene was ligated with a pETDuet-1 vector, was prepared by performing PCR using the pETDado as a template, primers represented by SEQ ID NO: 9 and SEQ ID NO: 10 and KOD-Plus-Mutagenesis Kit (Toyobo). An expression bacterial body of E. coli transduced with a gene encoding Ado-F82Y was prepared by the same process as Preparation Example 1 using the pETDado-F82Y.

[Preparation Example of Transformant 3]

The ado-F82W gene (SEQ ID NO: 11), encoding the oxidase Ado-F82W having an amino acid sequence represented by SEQ ID NO: 3, was prepared by chemical synthesis. Specifically, the pETDado-F82W, in which the ado-F82W gene was ligated with a pETDuet-1 vector, was prepared by performing PCR using the pETDado as a template, primers represented by SEQ ID NO: 12 and SEQ ID NO: 13 and KOD-Plus-Mutagenesis Kit (Toyobo). An expression bacterial body of E. coli transduced with a gene encoding Ado-F82W was prepared by the same process as Preparation Example 1 using the pETDado-F82W.

[Preparation Example of Transformant 4]

The genes (SEQ ID NO: 14), encoding the oxidases Ado-F82Y-V332X-F333X-F334X having an amino acid sequence represented by SEQ ID NO: 4 in which an amino acid residue at at least one of the 332nd, 333rd or 334th position was randomly modified in addition to an amino acid residue at the 82nd position, was prepared.

Specifically, the plasmids pETDado-F82Y-V332X-F333X-F334X, in which the ado-F82Y-332X-F333X-F334X genes were ligated with a pETDuet-1 vector, were prepared by performing PCR using the plasmid pETDado-F82Y as a template, a mix primer represented by SEQ ID NO: 15, a primer represented by SEQ ID NO: 16 and KOD-Plus-Mutagenesis Kit (Toyobo). The plasmids were transduced into E. coli BL21 Star (DE3) (Thermo Fisher) by a heat shock method, thereby obtaining an expression bacterial body of E. coli transduced with genes encoding Ado-F82Y-332X-F333X-F334X.

[Example 1]

A reaction solution (250 μl) containing 40 mg/ml (final concentration, based on the weight of wet bacterial body) of the expression bacterial body of E. coli obtained in each of Preparation Examples 1-3, 10 mM of ferulic acid (following compound) as a substrate compound, 10% (v/v) of dimethylsulfoxide and 100 mM of Tris-HCl buffer (pH 9.5) was prepared. The reaction solution was caused to react for 18 hours while shaking at 30°C.

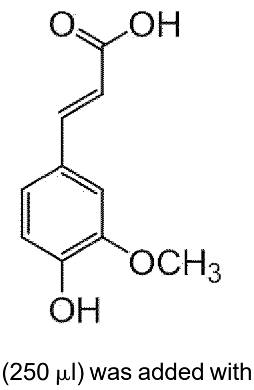

After the reaction, the reaction solution (250 μl) was added with 5N HCl (5 μl) and ethanol (250 μl) to prepare a sample for HPLC analysis. The HPLC analysis was performed using Prominence LC-20 system (Shimadzu) and XTerra MS C18 IS (Waters) as a column.

In the analysis, 0.1% formic acid aqueous solution (liquid A) and methanol (liquid B) were used as the developing solvent. The developing solvent was allowed to flow at a rate of 0.5 ml/min at a mixture ratio of 95% (liquid A) and 5% (liquid B) over a time period from 0 min to 3 min. Thereafter, the ratio of the liquid B in the developing solvent was increased in a linear manner up to 40% over a time period from 3 min to 4 min; up to 80% over a time period from 4 min to 14 min; and up to 100% over a time period from 14 min to 15 min. After flowing the developing solvent at a ratio of liquid B being 100% over a time period from 15 min to 18 min, the ratio of the liquid B in the developing solvent was lowered in a linear manner down to 5% over a time period from 18 min to 19 min, and the developing solvent were allowed to flow at a ratio of liquid B being 5% over a time period from 19 min to 22 min, thereby eluting the compound.

As the results of the HPLC analysis, a peak corresponding to the product was detected at a retention time of 10.5 min in the case of the reaction solutions containing the expression bacterial body of E. coli obtained in the Preparation

Example 2 and the Preparation Example 3. The retention time of the peak of the product coincided with the retention time of the peak of authentic vanillin. In the case of the reaction solution containing the expression bacterial body of E. coli obtained in the Preparation Example 1, no peak corresponding to the product was detected at a retention time of 10.5 min.

The results of the measurement of absorbing spectrum of the product are shown in FIG.1. As shown in FIG. 1, the absorbing spectrum of the product detected after the reaction coincided with the absorbing spectrum of authentic vanillin, and therefore the product was identified as vanillin.

The results of the quantification by HPLC for vanillin as the product (n=3) are shown in FIG. 2.

As shown in FIG. 2, while vanillin was not produced when Ado was used in the enzymatic reaction, vanillin was produced by 0.023 mM when Ado-F82Y was used in the enzymatic reaction, and by 0.007 mM when Ado-F82W was used in the enzymatic reaction.

[Example 2]

A reaction solution was prepared in the same manner as Example 1, except that the substrate compound was changed to ethyl ferulate (following compound), caused to react for 18 hours while shaking at 30°C, and subjected to the HPLC analysis.

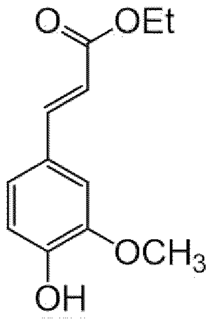

As the results of the HPLC analysis, a peak corresponding to the product was detected at a retention time of 10.5 min in the reaction solution containing the expression bacterial body of E. coli obtained in the Preparation Example 1, Preparation Example 2 and Preparation Example 3, respectively. The retention time of the peak of the product coincided with the retention time of the peak of authentic vanillin.

The results of the quantification by HPLC for vanillin as the product (n=3) are shown in FIG. 3. As shown in FIG. 3, vanillin was produced by 0.075 mM when Ado was used in the enzymatic reaction, by 0.376 mM when Ado-F82Y was used in the enzymatic reaction, and by 0.234 mM when Ado-F82W was used in the enzymatic reaction.

[Example 3]

A reaction solution was prepared in the same manner as Example 1, except that the substrate compound was changed to 4-vinyl guaiacol (following compound), caused to react for 2 hours while shaking at 30°C, and subjected to the HPLC analysis.

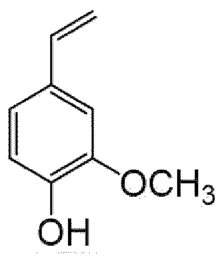

As a result of the HPLC analysis, a peak corresponding to the product was detected at a retention time of 10.5 min in the reaction solution containing the expression bacterial body of E. coli obtained in the Preparation Example 1, Preparation Example 2 and Preparation Example 3, respectively. The retention time of the peak of the product coincided with the retention time of the peak of authentic vanillin.

The results of the quantification by HPLC for vanillin as the product (n=3) are shown in FIG. 4. As shown in FIG. 4, vanillin was produced by 1.032 mM when Ado was used in the enzymatic reaction, by 5.408 mM when Ado-F82Y was used

in the enzymatic reaction, and by 1.565 mM when Ado-F82W was used in the enzymatic reaction.

[Example 4]

A reaction solution was prepared in the same manner as Example 1, except that the substrate compound was changed to isoeugenol (following compound), caused to react for 2 hours while shaking at 30°C, and subjected to the HPLC analysis.

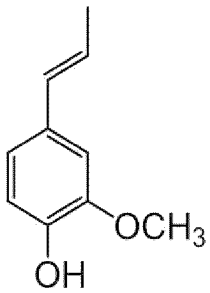

As the results of the HPLC analysis, a peak corresponding to the product was detected at a retention time of 10.5 min in the reaction solution containing the expression bacterial body of E. coli obtained in the Preparation Example 1, Preparation Example 2 and Preparation Example 3, respectively. The retention time of the peak of the product coincided with the retention time of the peak of authentic vanillin.

The results of the quantification by HPLC for vanillin as the product (n=3) are shown in FIG. 5. As shown in FIG. 5, vanillin was produced by 1.934 mM when Ado was used in the enzymatic reaction, by 5.105 mM when Ado-F82Y was used in the enzymatic reaction, and by 2.602 mM when Ado-F82W was used in the enzymatic reaction.

From the results of the foregoing Examples, it is proved that the compound represented by Formula (2) can be produced from the compound represented by Formula (1) by a single step of enzymatic reaction, using an enzyme having an activity to cleave the carbon-carbon double bond in the compound represented by Formula (1).

Further, it is proved that the amount of production of the compound represented by Formula (2) is greater in the case in which the enzyme having an amino acid sequence represented by SEQ ID NO: 2 or SEQ ID NO: 3 is used, as compared with the case in which the enzyme having an amino acid sequence represented by SEQ ID NO: 1 is used.

[Example 5]

The expression bacterial body of E. coli prepared in Preparation Example 4 was inoculated in the form of streaks into an LB medium containing ampicillin (50 μg/ml), isopropyl-β-D-thiogalactopyranoside (0.1 mM) and $FeCl_2$ (1 mM), and cultured for 24 hours at 20°C.

A reaction solution (50 μl) containing approximately 80 mg/ml (final concentration, based on the weight of wet bacterial body) of the expression bacterial body of E. coli, ferulic acid (10 mM), dimethylsulfoxide (10% v/v) and 100 mM of Tris-HCl buffer (pH 9.5) was prepared. The reaction solution was caused to react for 24 hours while shaking at 30°C.

Further, the reaction solution was analyzed by thin-layer chromatography (Aluminum TLC plate, silica gel coated with fluorescent indicator F254, Merck), and a superior recombinant E. Coli with a greater production amount of vanillin than Ado-F82Y recombinant E. coli was selected (M13, M26, M40, M41, M56, M57, M69, M70 and M79).

Subsequently, the superior recombinant E. coli was inoculated into an LB medium containing ampicillin (50 μg/ml) and cultured for 6 hours at 30°C. Thereafter, isopropyl-β-D-thiogalactopyranoside was added at a final concentration of 0.1 mM and $FeCl_2$ was added at a final concentration of 1 mM, and cultured for 15 hours at 16°C to induce the expression of gene. The bacterial body was collected and washed with a phosphate buffer solution (137 mM NaCl, 2.7 mM KCl, 10 mM $Na_2HPO_4$, 2 mM $KH_2PO_4$, pH 7.4) and cryopreserved. A reaction solution (250 μl) containing 40 mg/ml (final concentration, based on the weight of wet bacterial body) of the superior recombinant E. coli, ferulic acid (10 mM), dimethylsulfoxide (10% v/v) and Tris-HCl buffer (100 mM, pH 9.5) was prepared. The reaction solution was caused to react for 24 hours while shaking at 30°C. Thereafter, the reaction solution (250 μl) was added with 5N HCl (5 μl) and ethanol (250 μl) to prepare a sample for HPLC analysis. The HPLC analysis was performed in the same manner as Example 1. For the purpose of comparison, the HPLC analysis was performed on a sample with Ado-F82Y. The results are shown in FIG. 6.

As shown in FIG. 6, the samples prepared from the expression bacterial body of E. coli transduced with the genes encoding Ado-F82Y-V332X-F333X-F334X can produce vanillin by an amount of 0.2 mM or more.

The amino acid sequence of the modified enzyme M40 with a remarkably high conversion rate from ferulic acid to vanillin was analyzed. As a result, the amino acid residue at the 332nd position was an arginine residue, the amino acid residue at the 333rd position was isoleucine residue, and the amino acid residue at the 334th position was a leucine

residue.

The reason why the modified enzyme M40 exhibits a high rate of conversion is thought to be that, for example, an electrostatic interaction is formed between the carboxy group of ferulic acid and the 332nd arginine residue, being located close to the carboxy group, thereby increasing the affinity of the enzyme with respect to ferulic acid.

[Example 6]

A reaction solution was prepared in the same manner as Example 5, using the expression bacterial body of E. coli prepared in Preparation Example 4. The reaction solution was shaken for 24 hours at 30°C. Further, the reaction solution was analyzed by thin-layer chromatography, and a superior recombinant E. coli with a greater production amount of vanillin than Ado-F82Y recombinant E. coli was selected. Then, a reaction solution was prepared in the same manner as Example 5, using the selected superior recombinant E. coli. The reaction solution was allowed to react for 24 hours while shaking at 30°C, and subjected to HPLC analysis. The results are shown in FIG. 7.

As shown in FIG. 7, the samples prepared from the expression bacterial body of E. coli transduced with the genes encoding Ado-F82Y-V332X-F333X-F334X include M258, a sample that can produce vanillin by an amount of over 5 mM.

The amino acid sequence of the modified enzymes with a remarkably high conversion rate from ferulic acid to vanillin was analyzed. As a result, the amino acid residues at the 332nd position, 333rd position and 334th position were substituted by various kinds of amino acid residues. The results are shown in Table 1. The results show that modification to these amino acid residues is effective to increase the activity of Ado with respect to ferulic acid.

Table 1

| Protein | Amino acid substitutions | | |
|---|---|---|---|
| | 332 | 333 | 334 |
| Ado WT, F82Y | V | F | F |
| M40 | R | I | L |
| M57 | R | E | S |
| M129 | R | S | R |
| M166 | R | H | C |
| M246 | T | G | F |
| M253 | K | G | I |
| M258 | R | F | R |
| M265 | R | V | L |
| M270 | R | S | R |
| M324 | R | G | G |
| M447 | R | L | N |
| M458 | K | G | M |

[Example 7]

A reaction solution was prepared using the expression bacterial body of the modified enzyme M258, which expresses a high degree of activity in Example 6, in the same manner as Example 5 except that the substrate compound was changed from ferulic acid to p-coumaric acid (following compound) of the same amount. The reaction solution was allowed to react for 24 hours while shaking at 30°C, and subjected to HPLC analysis.

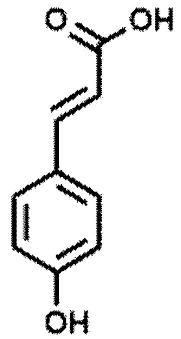

In the results of HPLC analysis, a peak corresponding to the product was detected at a retention time of 6.16 min in the reaction solution containing the expression bacterial body of M258. The retention time of the peak of the product coincided with a peak of authentic p-hydroxy benzaldehyde (following compound).

The results of the quantification by HPLC for p-hydroxy benzaldehyde as the product (n=3) are shown in FIG. 8. As shown in FIG. 8, p-hydroxy benzaldehyde was produced by 0.43 mM when M258 was used in the enzymatic reaction.

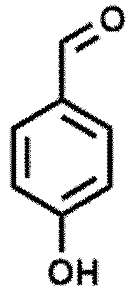

[Example 8]

A reaction solution was prepared using the expression bacterial body of the modified enzyme M258, which expresses a high degree of activity in Example 6, in the same manner as Example 5 except that the substrate compound was changed from ferulic acid to sinapic acid (following compound) of the same amount. The reaction solution was allowed to react for 24 hours while shaking at 30°C, and subjected to HPLC analysis.

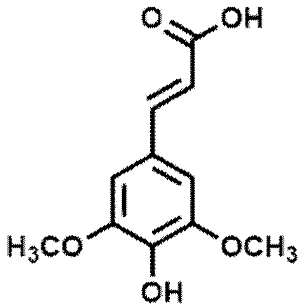

In the results of HPLC analysis, a peak corresponding to the product was detected at a retention time of 11.5 min in the reaction solution containing the expression bacterial body of M258. The retention time of the peak of the product coincided with a peak of authentic syringaldehyde (following compound).

The results of the quantification by HPLC for syringaldehyde as the product (n=3) are shown in FIG. 9. As shown in FIG. 9, syringaldehyde was produced by 8.6 mM when M258 was used in the enzymatic reaction.

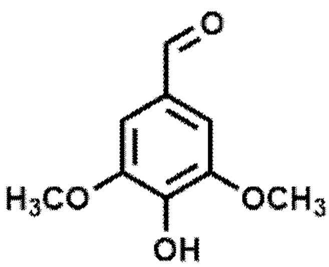

[Example 9]

A reaction solution was prepared using the expression bacterial body of the modified enzyme M258, which expresses a high degree of activity in Example 6, in the same manner as Example 5 except that the substrate compound was changed from ferulic acid to coniferyl alcohol (following compound) of the same amount. The reaction solution was allowed to react for 24 hours while shaking at 30°C, and subjected to HPLC analysis.

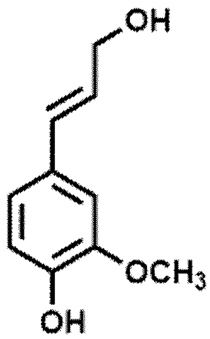

In the results of HPLC analysis, a peak corresponding to the product was detected at a retention time of 10.6 min in the reaction solution containing the expression bacterial body of M258. The retention time of the peak of the product coincided with a peak of authentic vanillin.

The results of the quantification by HPLC for vanillin as the product (n=3) are shown in FIG. 10. As shown in FIG. 10, vanillin was produced by 3.4 mM when M258 was used in the enzymatic reaction.

[Example 10]

Vanillin was produced from ferulic acid using the expression bacterial body of the modified enzyme M258, which expresses a high degree of activity in Example 6, while changing the reaction conditions.

Specifically, a reaction solution (250 μl) containing 80 mg/ml (final concentration, based on the weight of wet bacterial body) of the expression bacterial body of modified enzyme M258, ferulic acid (10 mM, 20mM or 30 mM), butyl acetate (10 (v/v)) and Tris-HCl buffer (100 mM, pH 10.5) was prepared. The reaction solution was caused to react for 48 hours at 30°C, and subjected to HPLC analysis.

The results of the quantification by HPLC for vanillin as the product (n=3) are shown in FIG. 11. As shown in FIG. 11, the concentration of vanillin as the product was increased as the concentration of ferulic acid as the substrate material was increased, and vanillin was produced by an amount of 16.0 mM (2.44 g/L) from 30 mM of ferulic acid. The results suggest that the modified enzyme of the present disclosure will make it possible to produce vanillin by an amount of grams per liter scale.

The disclosure of Japanese Patent Application Nos. 2022-120869 and 2022-158419 is incorporated herein by reference in its entirety.

All publications, patent applications, and technical standards mentioned in the present specification are incorporated herein by reference to the same extent as if each individual publication, patent application, or technical standard was specifically and individually indicated to be incorporated by reference.

## Claims

**1.** A method of producing a phenolic compound, the method producing a compound represented by the following Formula (2) from a compound represented by the following Formula (1):

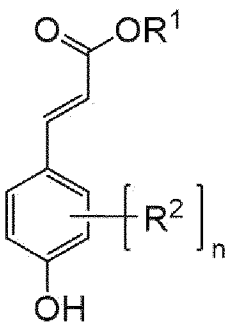

(1)

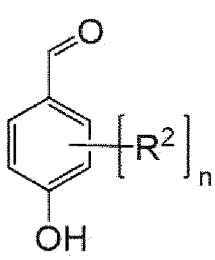

(2)

wherein, in the Formulas, $R^1$ represents a hydrogen atom or an alkyl group, each $R^2$ independently represents an alkoxy group, a hydroxy group, an alkyl group or a halogen atom, and n is a number from 0 to 4,
the method comprising a process of contacting the compound represented by Formula (1) with an enzyme,

the enzyme having an activity to cleave a carbon-carbon double bond in the compound represented by Formula (1), and

the method excluding a case in which the compound represented by Formula (1) is ferulic acid and the enzyme is a protein having an amino acid sequence represented by SEQ ID NO: 1.

**2.** The method of producing a phenolic compound according to claim 1, wherein the enzyme is a cleavage oxygenase that acts on a compound having a carbon-carbon double bond as a substrate.

**3.** The method of producing a phenolic compound according to claim 1, wherein the enzyme is a protein having an amino acid sequence that satisfies at least one condition selected from the following (a1) to (a6) and (b):

(a1) an amino acid residue at an 82nd position in an amino acid sequence represented by SEQ ID NO: 1 is an amino acid residue other than a phenylalanine residue;
(a2) an amino acid residue at a 124th position in the amino acid sequence represented by SEQ ID NO: 1 is an amino acid residue other than a tyrosine residue;
(a3) an amino acid residue at a 157th position in the amino acid sequence represented by SEQ ID NO: 1 is an amino acid residue other than a lysine residue;
(a4) an amino acid residue at a 332nd position in the amino acid sequence represented by SEQ ID NO: 1 is an amino acid residue other than a valine residue;
(a5) an amino acid residue at a 333rd position in the amino acid sequence represented by SEQ ID NO: 1 is an amino acid residue other than a phenylalanine residue;
(a6) an amino acid residue at a 334th position in the amino acid sequence represented by SEQ ID NO: 1 is an amino acid residue other than a phenylalanine residue; and
(b) having an amino acid sequence identity of 70% or more with respect to an amino acid sequence that satisfies a condition selected from (a1) to (a6), expect for the amino acid sequence represented by SEQ ID NO: 1.

**4.** The method of producing a phenolic compound according to claim 3, wherein the enzyme is a protein having an amino acid sequence that satisfies a condition of (a1) and at least one condition selected from (a2) to (a6), or a protein having an amino acid sequence identity of 70% or more with respect to the amino acid sequence.

**5.** The method of producing a phenolic compound according to claim 3, wherein in (a1), the amino acid residue at the 82nd portion is a tyrosine residue or a triptophan residue.

**6.** The method of producing a phenolic compound according to claim 3, wherein in (a4), (a5) or (a6), at least one amino acid residue at a position selected from the 332nd portion, the 333rd position or the 334 position is a polar amino acid residue.

**7.** The method of producing a phenolic compound according to claim 1, wherein the compound represented by Formula (1) is ferulic acid or a ferulic acid alkyl ester.

**8.** The method of producing a phenolic compound according to claim 1, wherein the compound represented by Formula (2) is vanillin or ethyl vanillin.

**9.** A method of producing a phenolic compound, the method producing a compound represented by the following Formula (4) from a compound represented by the following Formula (3):

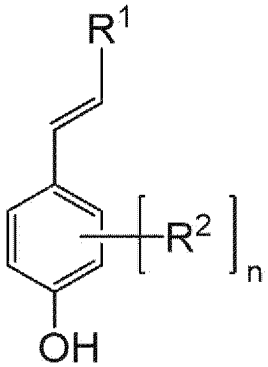
(3)

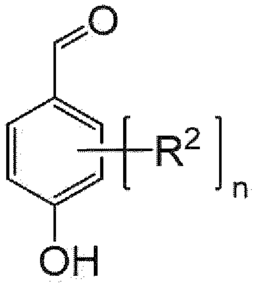
(4)

wherein, in the Formulas, $R^1$ represents a hydrogen atom, an alkyl group or $COOR^3$, each $R^2$ independently represents an alkoxy group, a hydroxy group, an alkyl group or a halogen atom, $R^3$ represents a hydrogen atom or an alkyl group, and n is a number from 0 to 4,
the method comprising a process of contacting the compound represented by Formula (3) with an enzyme, and the enzyme having an activity to cleave a carbon-carbon double bond in the compound represented by Formula (3) and being a protein having an amino acid sequence that satisfies at least one condition selected from the following (a1) to (a6) and (b):

(a1) an amino acid residue at an 82nd position in an amino acid sequence represented by SEQ ID NO: 1 is an amino acid residue other than a phenylalanine residue;
(a2) an amino acid residue at a 124th position in the amino acid sequence represented by SEQ ID NO: 1 is an amino acid residue other than a tyrosine residue;
(a3) an amino acid residue at a 157th position in the amino acid sequence represented by SEQ ID NO: 1 is an amino acid residue other than a lysine residue;
(a4) an amino acid residue at a 332nd position in the amino acid sequence represented by SEQ ID NO: 1 is an amino acid residue other than a valine residue;
(a5) an amino acid residue at a 333rd position in the amino acid sequence represented by SEQ ID NO: 1 is an amino acid residue other than a phenylalanine residue;
(a6) an amino acid residue at a 334th position in the amino acid sequence represented by SEQ ID NO: 1 is an amino acid residue other than a phenylalanine residue; and
(b) having an amino acid sequence identity of 70% or more with respect to an amino acid sequence that satisfies a condition selected from (a1) to (a6), expect for the amino acid sequence represented by SEQ ID NO: 1.

**10.** A modified enzyme, having an activity to cleave a carbon-carbon double bond in a phenolic compound, and being a protein having an amino acid sequence that satisfies at least one condition selected from the following (a1) to (a6) and (b):

(a1) an amino acid residue at an 82nd position in an amino acid sequence represented by SEQ ID NO: 1 is an amino acid residue other than a phenylalanine residue;
(a2) an amino acid residue at a 124th position in the amino acid sequence represented by SEQ ID NO: 1 is an amino acid residue other than a tyrosine residue;
(a3) an amino acid residue at a 157th position in the amino acid sequence represented by SEQ ID NO: 1 is an amino acid residue other than a lysine residue;
(a4) an amino acid residue at a 332nd position in the amino acid sequence represented by SEQ ID NO: 1 is an amino acid residue other than a valine residue;
(a5) an amino acid residue at a 333rd position in the amino acid sequence represented by SEQ ID NO: 1 is an amino acid residue other than a phenylalanine residue;
(a6) an amino acid residue at a 334th position in the amino acid sequence represented by SEQ ID NO: 1 is an amino acid residue other than a phenylalanine residue; and
(b) having an amino acid sequence identity of 70% or more with respect to an amino acid sequence that satisfies a condition selected from (a1) to (a6), expect for the amino acid sequence represented by SEQ ID NO: 1.

**11.** The modified enzyme according to claim 10, which is used for a method of producing a phenolic compound.

**12.** A composition, comprising the modified enzyme according to claim 10.

**13.** A polynucleotide, encoding the modified enzyme according to claim 10.

**14.** An expression vector, comprising the polynucleotide according to claim 13.

**15.** A transformant, comprising the expression vector according to claim 14.

FIG.1

(A)

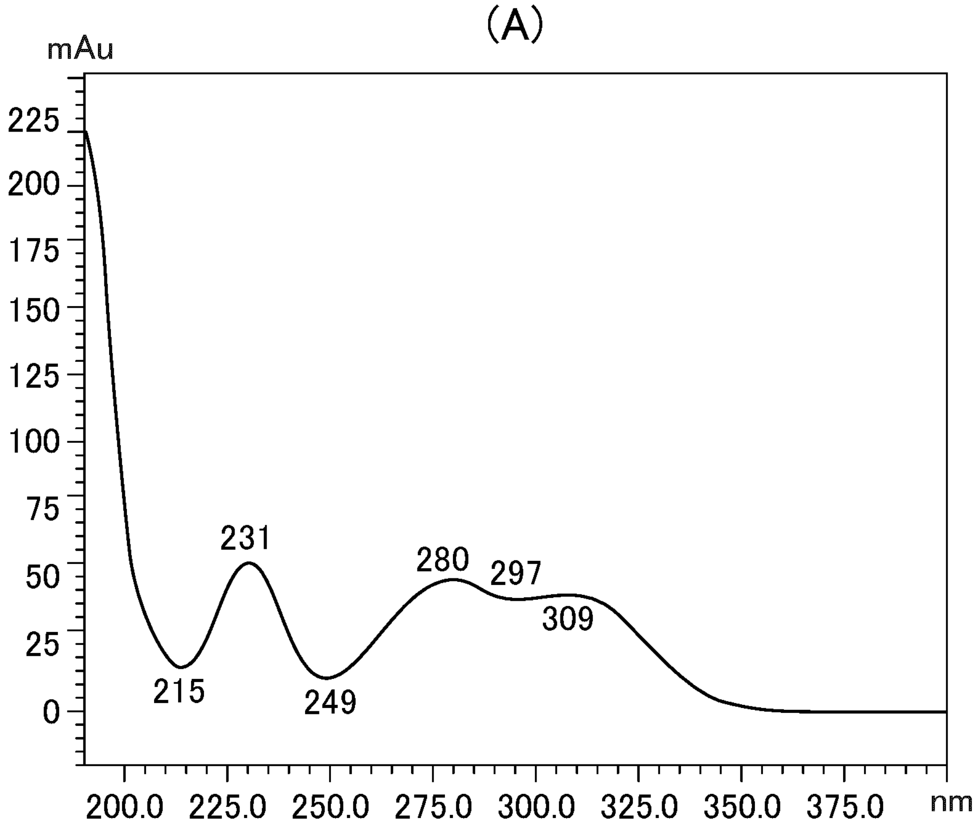
mAu
225
200
175
150
125
100
75
50
25
0
231
280
297
309
215
249
200.0
225.0
250.0
275.0
300.0
325.0
350.0
375.0
nm

(B)

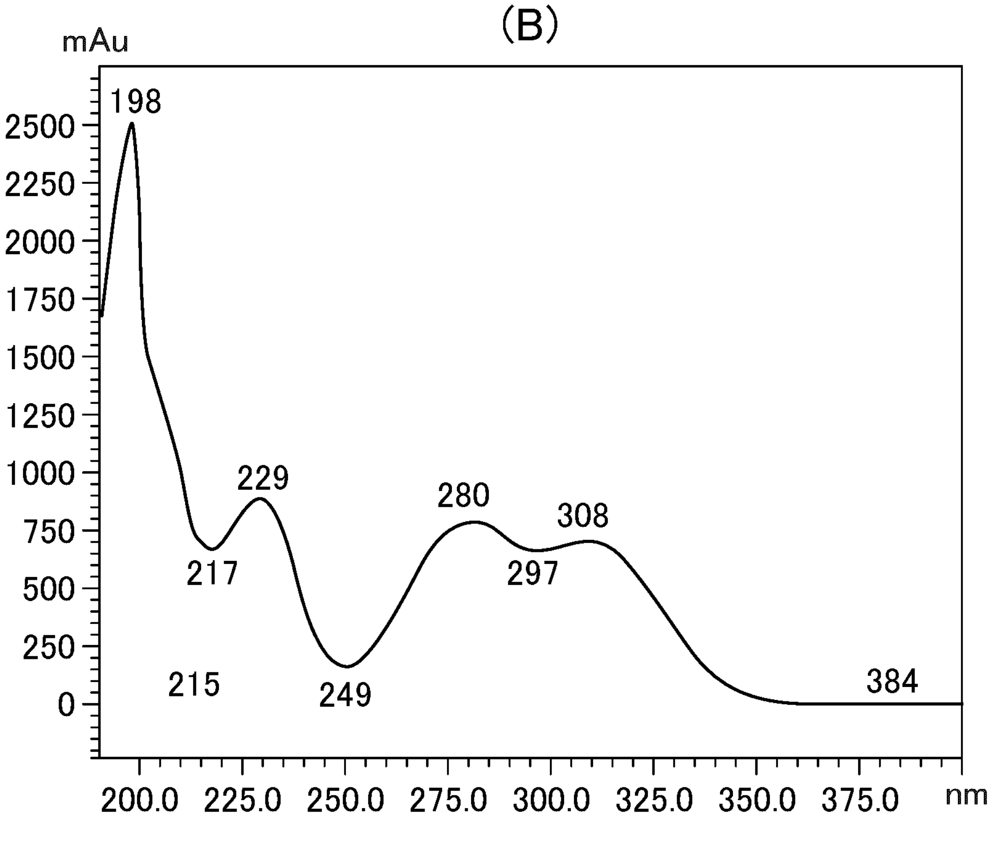
mAu
2500
2250
2000
1750
1500
1250
1000
750
500
250
0
198
229
280
308
217
297
215
249
384
200.0
225.0
250.0
275.0
300.0
325.0
350.0
375.0
nm

FIG.2

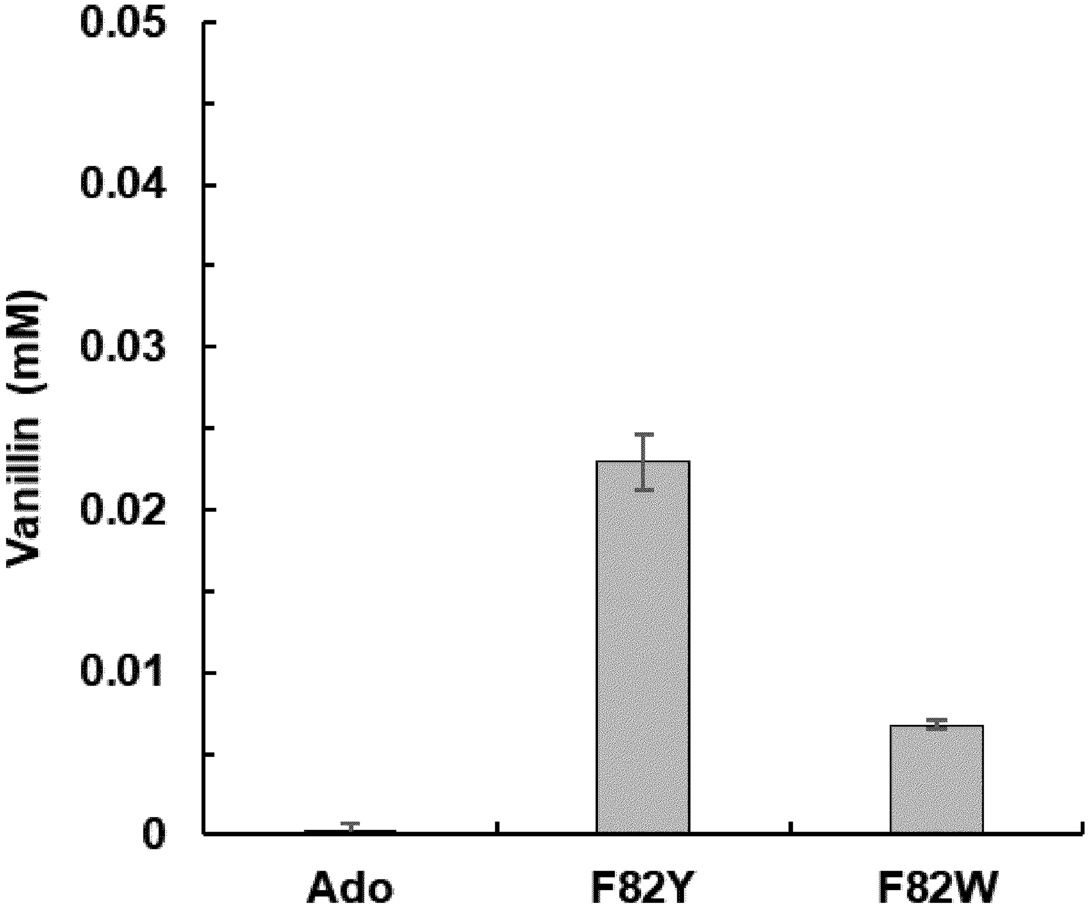

FIG.3

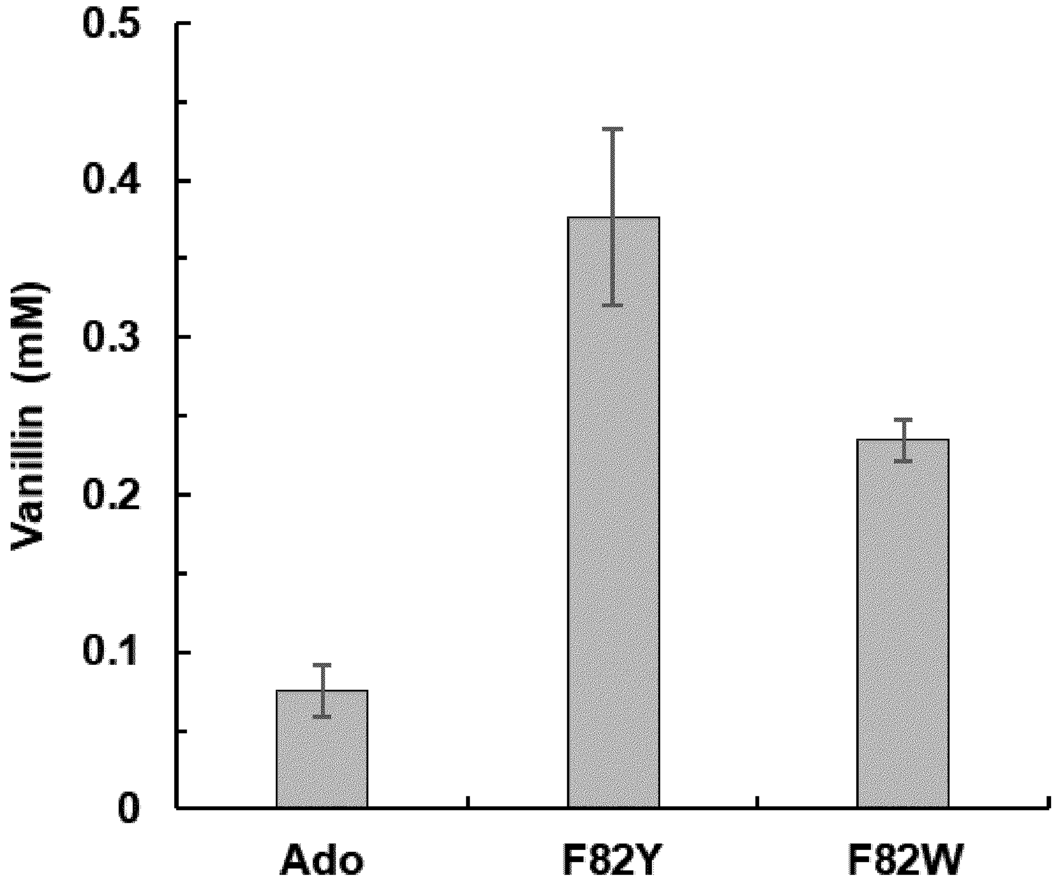

FIG.4

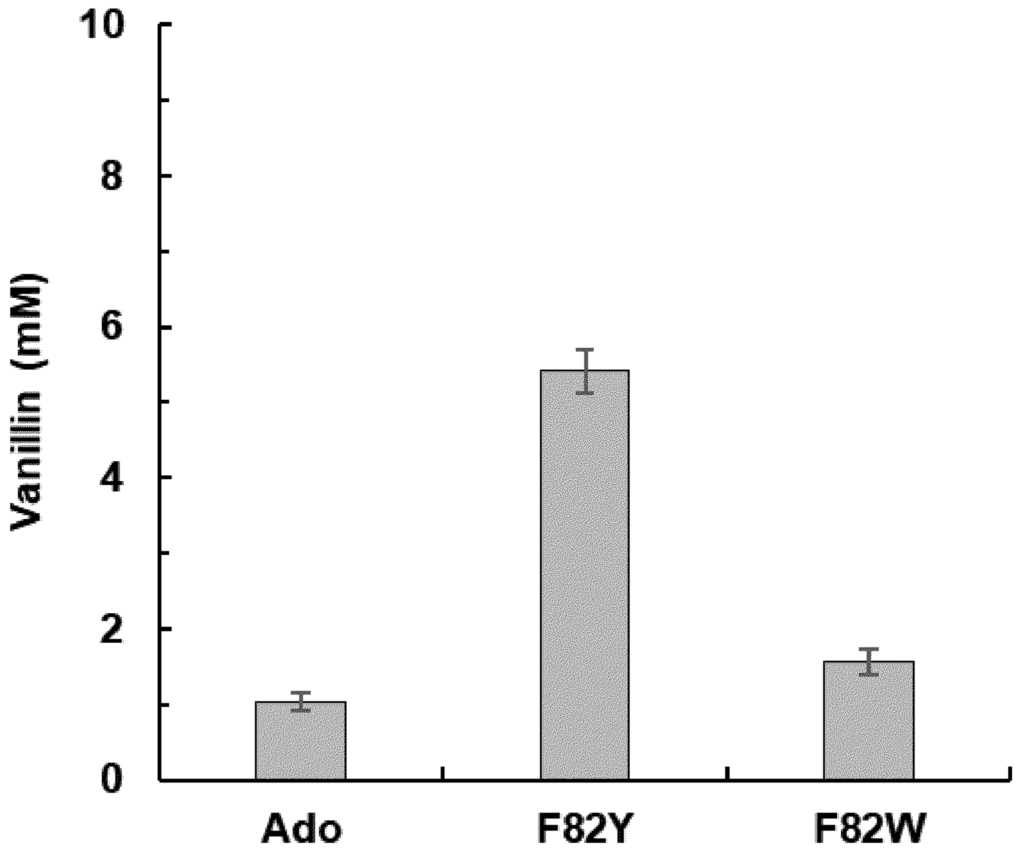

FIG.5

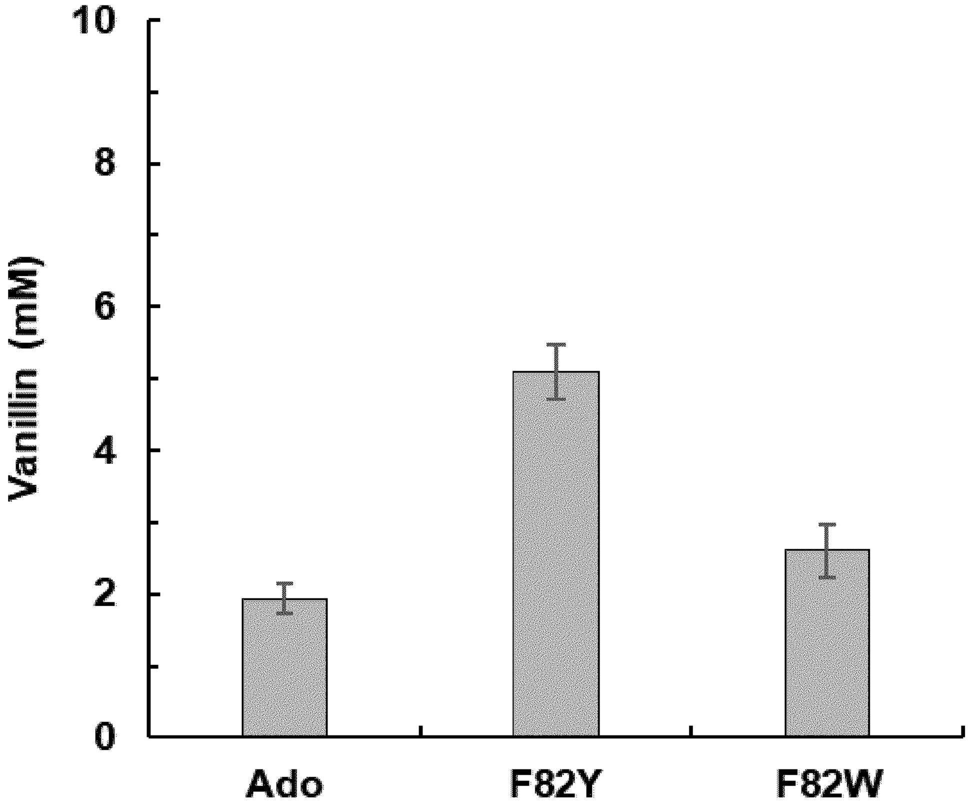

FIG.6

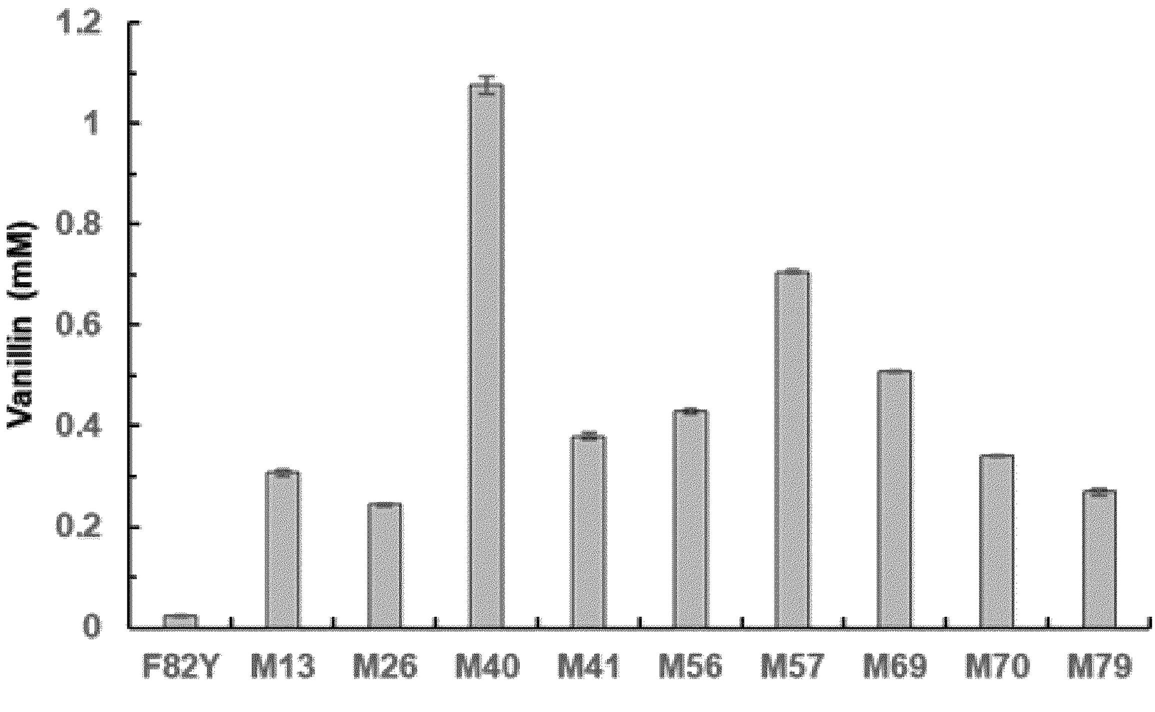
Vanillin (mM)
1.2
1
0.8
0.6
0.4
0.2
0
F82Y
M13
M26
M40
M41
M56
M57
M69
M70
M79

FIG.7

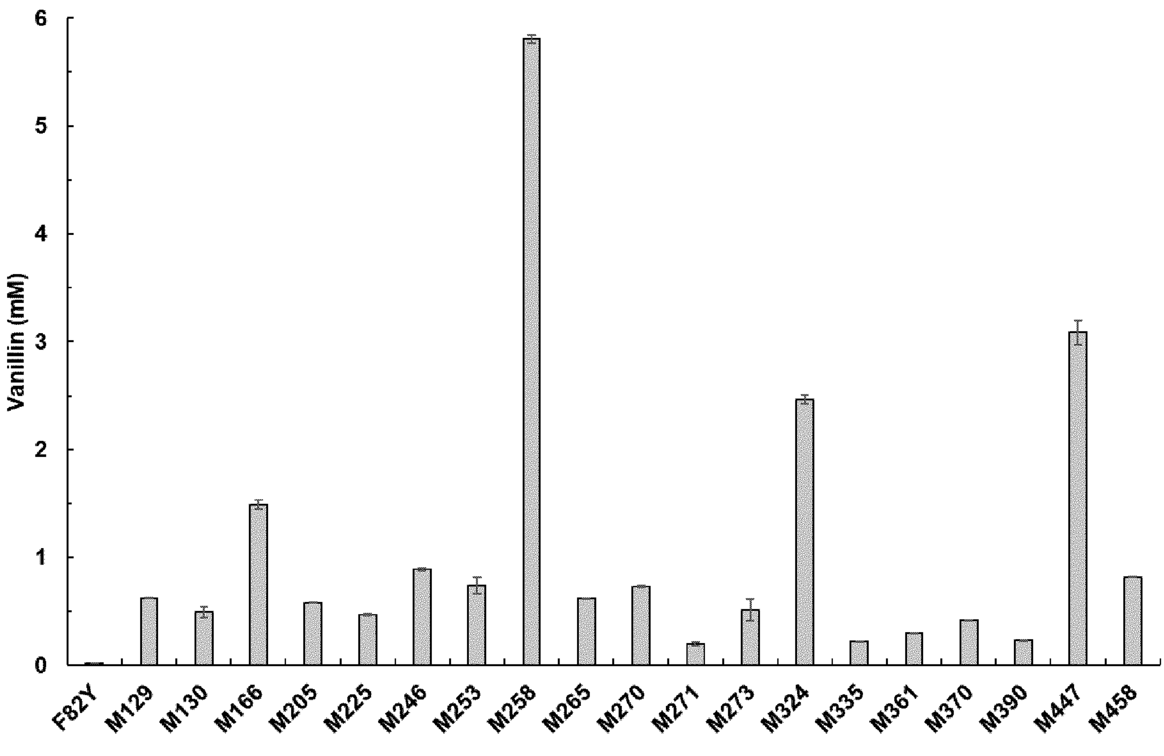
6
5
4
3
2
1
0
Vanillin (mM)
F82Y
M129
M130
M166
M205
M225
M246
M253
M258
M265
M270
M271
M273
M324
M335
M361
M370
M390
M447
M458

FIG.8

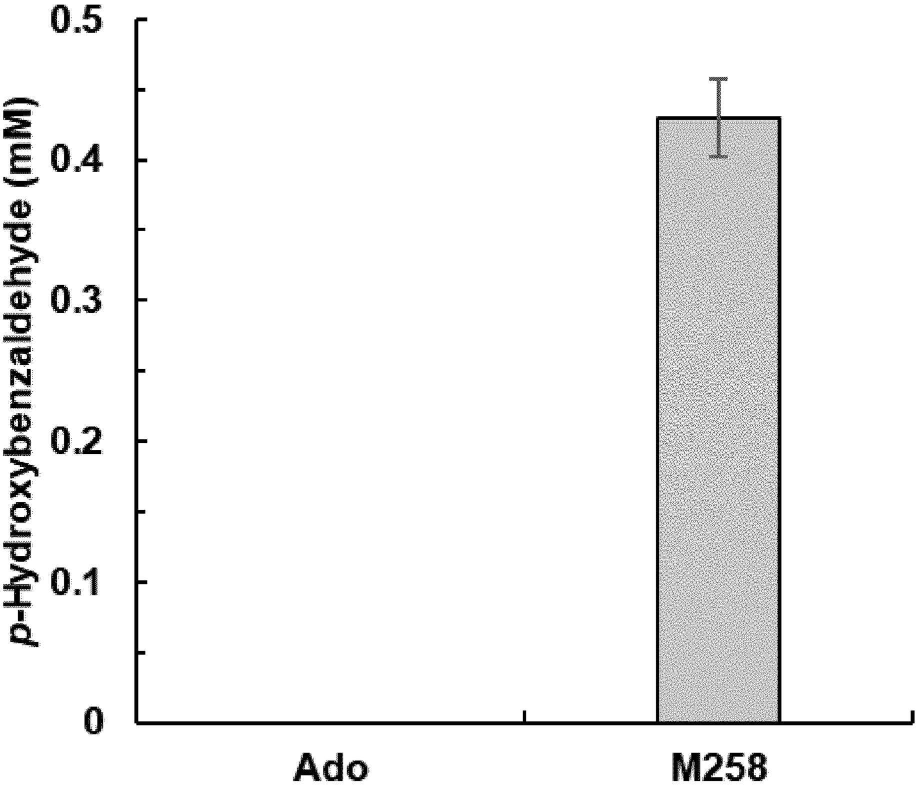

FIG.9

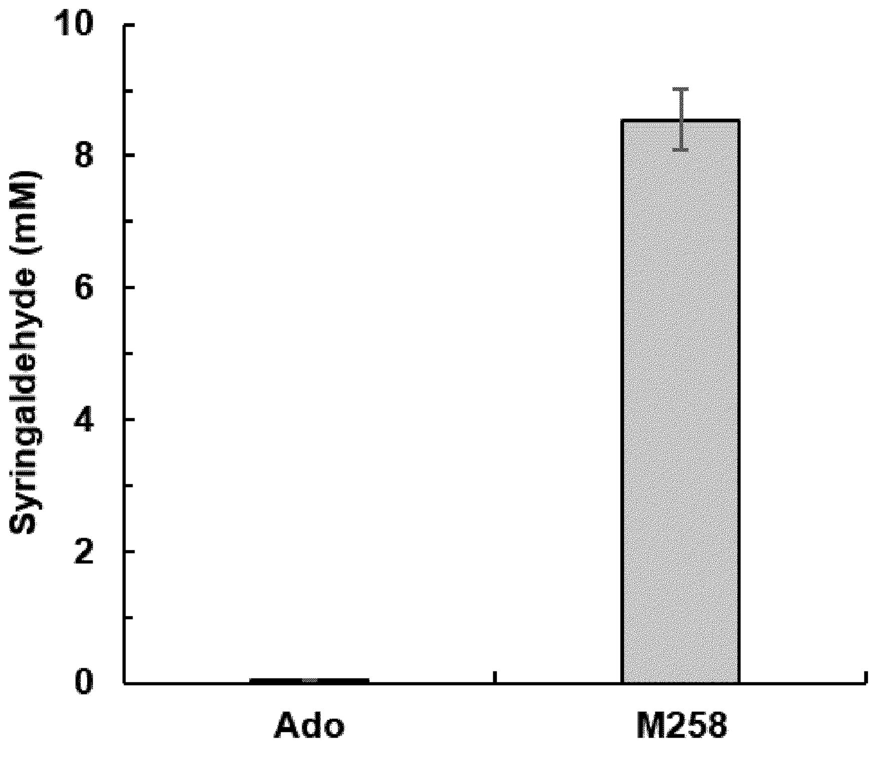

FIG.10

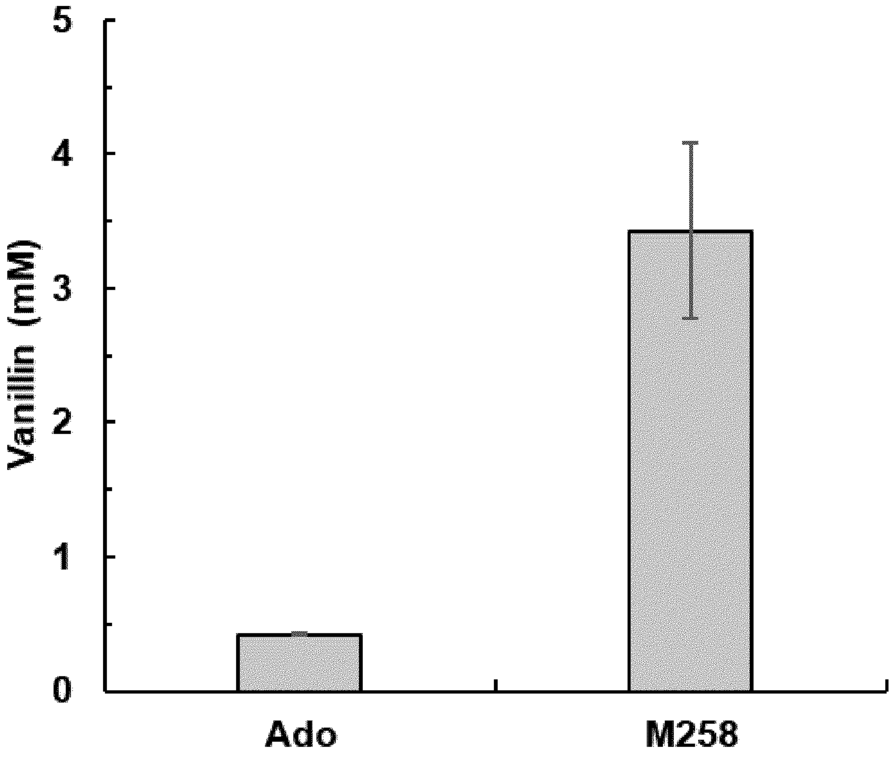

FIG.11

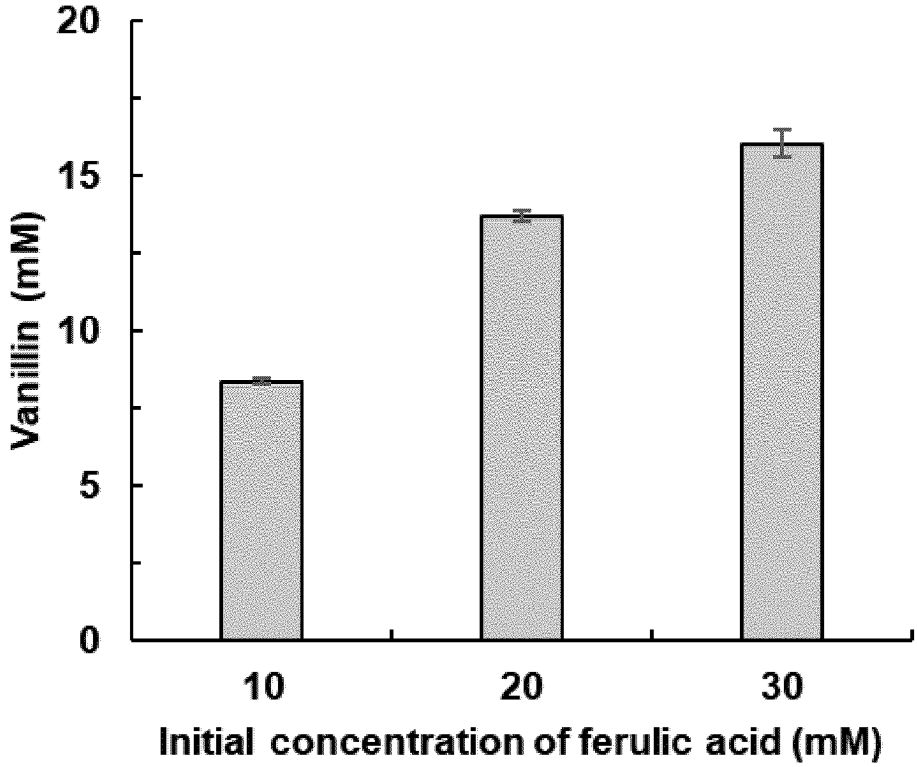
20
15
10
5
0
Vanillin (mM)
10
20
30
Initial concentration of ferulic acid (mM)

## INTERNATIONAL SEARCH REPORT

International application No.
**PCT/JP2023/027278**

### A. CLASSIFICATION OF SUBJECT MATTER

***C12N 15/53***(2006.01)i; ***C12N 1/15***(2006.01)i; ***C12N 1/19***(2006.01)i; ***C12N 1/21***(2006.01)i; ***C12N 5/10***(2006.01)i; ***C12N 9/02***(2006.01)i; ***C12N 9/04***(2006.01)i; ***C12N 15/63***(2006.01)i; ***C12P 7/22***(2006.01)i
FI: C12N15/53; C12N9/04 Z; C12N15/63 Z; C12N1/15; C12N1/19; C12N1/21; C12N5/10; C12N9/02; C12P7/22 ZNA

According to International Patent Classification (IPC) or to both national classification and IPC

### B. FIELDS SEARCHED

Minimum documentation searched (classification system followed by classification symbols)

C12N15/53; C12N1/15; C12N1/19; C12N1/21; C12N5/10; C12N9/02; C12N9/04; C12N15/63; C12P7/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq; UniProt/GeneSeq

### C. DOCUMENTS CONSIDERED TO BE RELEVANT

| Category* | Citation of document, with indication, where appropriate, of the relevant passages | Relevant to claim No. |
|---|---|---|
| X | GALLAGE, N. J. et al. Vanillin formation from ferulic acid in Vanilla planifolia is catalysed by a single enzyme. Nature Communications. 2014, vol. 5, no. 4037, pp. 1-14<br>abstract, fig. 10 | 1-8 |
| A | | 9-15 |
| X | CN 108715826 A (SHANGHAI JIAO TONG UNIVERSITY) 30 October 2018 (2018-10-30)<br>claims, examples, sequence tables | 1-8 |
| A | | 9-15 |
| X | NI, J. et al. A Coenzyme-Free Biocatalyst for the Value-Added Utilization of Lignin-Derived Aromatics. J Am Chem Soc. 2018, vol. 140, pp. 16001-16005<br>abstract, fig. 1 | 9-15 |
| A | | 1-8 |
| A | WO 2020/223417 A1 (CONAGEN INC.) 05 November 2020 (2020-11-05)<br>claims, examples | 9-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 September 2023** | **10 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/027278**

**C. DOCUMENTS CONSIDERED TO BE RELEVANT**

| Category* | Citation of document, with indication, where appropriate, of the relevant passages | Relevant to claim No. |
|---|---|---|
| A | WO 2020/223418 A1 (CONAGEN INC.) 05 November 2020 (2020-11-05) claims, examples | 8-15 |
| P, X | 坂本紗津記ほか, 3A03-05 炭素間二重結合を酸化開裂する酵素の機能改変とバニリン合成への応用, 第７４回日本生物工学会大会講演要旨集, 03 October 2022, p. 152 in particular, section "Method and results", (SAKAMOTO, Satsuki et al. 3A03-05 Mutagenesis of a C=C bond cleavage oxygenase and its use in vanillin synthesis.), non-official translation (Lecture Abstracts of 75th SBJ Annual Meeting) | 1-15 |

Form PCT/ISA/210 (second sheet) (January 2015)

**INTERNATIONAL SEARCH REPORT**

International application No.
**PCT/JP2023/027278**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   "In the form of an Annex C/ST.25 text file" above should be understood as "in ST.26 format".

Form PCT/ISA/210 (continuation of first sheet) (January 2015)

**INTERNATIONAL SEARCH REPORT**
**Information on patent family members**

International application No.
**PCT/JP2023/027278**

| Patent document cited in search report | Publication date (day/month/year) | Patent family member(s) | Publication date (day/month/year) |
|---|---|---|---|
| CN 108715826 A | 30 October 2018 | (Family: none) | |
| WO 2020/223417 A1 | 05 November 2020 | EP 3963086 A1<br>claims, examples<br>US 2022/0112525 A1 | |
| WO 2020/223418 A1 | 05 November 2020 | EP 3963078 A2<br>claims, examples<br>US 2022/0112526 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2022120869 A
- JP 2022158419 A

### Non-patent literature cited in the description

- *J Am Chem Soc*, 2018, vol. 140, 16001-16005
- *ChemBioChem*, 2014, vol. 15, 2248-2254
- *Biotechnol Lett*, 2005, vol. 27, 1829-1832
- *Nat Commun*, 2014, vol. 5, 4037